# EUROPEAN PATENT SPECIFICATION

(11) **EP 0 730 570 B1**
(45) Date of publication and mention of the grant of the patent: **19.04.2000**
(21) Application number: 95901697.3
(22) Date of filing: 31.10.1994
(51) Int. Cl.: C07C 215/28, C07C 301/02, C07D 301/02, C07D 303/36, C07C 221/00, C07C 223/02, C07C 269/04, C07C 271/20, C07C 213/08

(54) **METHOD FOR MAKING INTERMEDIATES USEFUL IN SYNTHESIS OF RETROVIRAL PROTEASE INHIBITORS**
VERFAHREN ZUR HERSTELLUNG VON ZWISCHENPRODUKTEN, DIE FÜR DIE HERSTELLUNG VON RETROVIRAL-PROTEASE HEMMENDEN VERBINDUNGEN VERWENDBAR SIND
PROCEDE DE PRODUCTION D'INTERMEDIAIRES UTILES DANS LA SYNTHESE D'INHIBITEURS DE PROTEASES RETROVIRALES

(30) Priority: 23.11.1993 US 156498
(43) Date of publication of application: 11.09.1996
(60) Divisional application: 98103779.9
(73) Proprietor: G.D. SEARLE & CO., Chicago IL 60680-5110 (US)
(72) Inventor: NG, John, S., Chicago, IL 60616 (US); PRZYBYLA, Claire, A., Des Plaines, IL 60016 (US); MUELLER, Richard, A., Glencoe, IL 60022 (US); VASQUEZ, Michael, L., Gurnee, IL 60031 (US); GETMAN, Daniel, P., Chesterfield, MO 63017 (US); FRESKOS, John, J., Clayton, MO 63105 (US); DECRESCENZO, Gary, A., St. Peters, MO 63376 (US); BERTENSHAW, Deborah, E., Brentwood, MO 63144 (US); HEINTZ, Robert, M., Ballwin, MO 63021 (US); ZHANG, Suhong, Des Plaines, IL 60016 (US); LIU, Chin, Northbrook, IL 60062 (US); LANEMAN, Scott, A., Vernon Hills, IL 60061 (US)
(74) Representative: Beil, Hans Christoph, Dr.
(86) International application number: US9412201
(87) International publication number: WO9514653

(56) References cited:
- EP-A- 0 288 764
- WO-A-92/14703
- WO-A-93/13066
- ANGEWANDTE CHEMIE. INTERNATIONAL EDITION, vol.26, no.11, November 1987, WEINHEIM DE pages 1141 - 3 M.T. REETZ ET. AL. 'Stereoselective Synthesis of beta-Amino alcohols from Optically Active alpha-Amino Acids.'
- JOURNAL OF ORGANIC CHEMISTRY., vol.50, no.23, 15 November 1985, EASTON US pages 4615 - 24 B.E. EVANS ET. AL. 'A Stereocontrolled Synthesis of Hydroxyethylene Dipeptide Isosteres Using Novel, chiral Aminoalkyl Epoxides and gamma-(Aminoalkyl) gamma-Lactones.'
- TETRAHEDRON LETTERS., vol.30, no.40, 1989, OXFORD GB pages 5425 - 8 M. T. REETZ ET. AL. 'Protective Group Tuning in the Stereoselective Conversion of alpha-Amino Aldehydes into Aminoalkyl Epoxides.'
- SYNLETT, no.9, September 1991, STUTTGART DE pages 631 - 2 T. J. MICHNICK ET. AL. '(Bromomethyl)lithium. Efficient in situ Reactions.'

## Description

### BACKGROUND OF THE INVENTION

### 1. Field of the Invention

Synthesis of many HIV protease inhibitors containing a hydroxyethylamine or hydroxyethylurea isostere include the amine opening of a key intermediate chiral epoxide. The synthesis of the key chiral epoxide requires a multi-step synthesis starting from L-phenylalanine and results in a low overall yield. The diastereoselectivity of the reduction step of the intermediate amino chloromethylketone is low and use of explosive diazomethane prevents the scale up of the method to multikilogram productions. The present invention relates to a method of preparing retroviral protease inhibitors and more particularly to a diastereoselective method of forming chiral intermediates for the preparation of urea containing hydroxyethylamine protease inhibitors.

### 2. Related Art

Roberts et al, Science, 248, 358 (1990), Krohn et al, J. Med. Chem. 344, 3340 (1991) and Getman, et al, J. Med. Chem., 346, 288 (1993) have previously reported synthesis of protease inhibitors containing the hydroxyethylamine or hydroxyethylurea isostere which include the opening of an epoxide generated in a multi-step synthesis starting from an amino acid. These methods also contain steps which include diazomethane and the reduction of an amino chloromethyl ketone intermediate to an amino alcohol prior to formation of the epoxide. The overall yield of these syntheses are low and the use of explosive diazomethane additionally prevents such methods from being commercially acceptable.

Tinker et al U.S. Patent No. 4,268,688 discloses a catalytic process for the asymmetric hydroformylation to prepare optically active aldehydes from unsaturated olefins. Similarly, Reetz et al U.S. Patent No. 4,990,669 discloses the formation of optically active alpha amino aldehydes through the reduction of alpha amino carboxylic acids or their esters with lithium aluminum hydride followed by oxidation of the resulting protected beta amino alcohol by dimethyl sulfoxide/oxalyl chloride or chromium trioxide/pyridine. Alternatively, protected alpha amino carboxylic acids or esters thereof can be reduced with diisobutylaluminum hydride to form the protected amino aldehydes.

THL, Vol. 30, No. 40, pp. 5425-5428 (1989) disclose a method for the stereoselective conversion of α-amino aldehydes into aminoalkyl epoxides using sulfonium and arsenium ylides.

The J. Org. Chem. 1985, 50, pp. 4615-4625 is concerned with the preparation of hydroxy alkylene dipeptide isosters using aminoalkyl epoxides and γ-lactones. Thereby, the aminoalkyl epoxides are prepared by reacting a respective aldehyde with a sulfonium ylide.

The reference Synlett, No. 9, pp 631-3 (1991) describes the preparation of (bromomethyl)-lithium and its usefulness in the preparation of epoxides using aldehydes or ketones.

EP A 0 288 764 discloses a method to form N,N-di-benzylphenylalanine and to reduce the same with LiAlH₄ to form respective N,N-dibenzylphenylalaninol.

WO-A-93 13066 describes the reaction of phenylalaninol with benzylbromide to form N,N-dibenzylphenylalaninol which can be alkylated.

Reetz et al (Tet. Lett., 30, 5425 (1989) disclosed the use of sulfonium and arsonium ylides and their reactions of protected α-amino aldehydes to form aninoalkyl epoxides. This method suffers from the use of highly toxic arsonium compounds or the use of combination of sodium hydride and dimethyl sulfoxide which is extremely hazardous in large scale. (Sodium hydride and DMSO are incompatible: Sax, N.I., "Dangerous Properties of Industrial Materials", 6th Ed., Van Nostrand Reinhold Co., 1984, p. 433. Violent explosions have been reported on the reaction of sodium hydride and excess DMSO, "Handbook of Reactive Chemical Hazards", 3rd Ed., Butterworths, 1985, p. 295. Matteson et al Synlett., 1991, 631 reported the addition of chloromethylithium or bromomethylithium to racemic aldehydes.

### SUMMARY OF THE INVENTION

Human immunodeficiency virus (HIV), the causative agent of acquired immunodeficiency syndrome (AIDS), encodes three enzymes, including the well-characterized proteinase belonging to the aspartic proteinase family, the HIV protease. Inhibition of this enzyme is regarded as a promising approach for treating AIDS. One potential strategy for inhibitor design involves the introduction of hydroxyethylene transition-state analogs into inhibitors. Inhibitors adapting the hydroxyethylamine or hydroxyethylurea isostere are found to be highly potent inhibitors of HIV proteases. Despite the potential clinical importance of these compounds, previously there were no satisfactory synthesis which could be readily and safely scaled up to prepare large kilogram quantities of such inhibitors needed for development and clinical studies. This invention provides an efficient synthesis of intermediates which are readily amenable to the large scale preparation of hydroxyethylureabased chiral HIV protease inhibitors.

Specifically, the method includes preparing a chiral diaminopropanol from a chiral alpha amino aldehyde via a diastereoselective epoxide or a stereospecific cyanohydrin.

### DETAILED DESCRIPTION OF THE INVENTION

This invention relates to a method that allows the preparation of commercial quantities of compounds of the formula 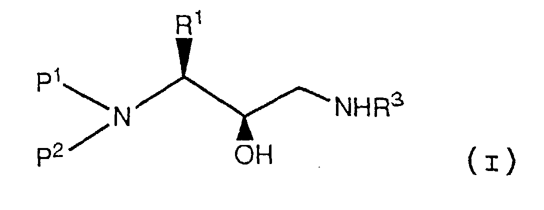 which are useful intermediates in the preparation of HIB protease inhibitors,
wherein R¹ is selected from alkyl, aryl, cycloalkyl, cycloalkylalkyl and arylalkyl, which are optionally substituted with a group selected from alkyl, halogen, NO₂, OR⁹ or SR⁹, where R⁹ represents hydrogen or alkyl; and
R³ represents hydrogen, alkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, heteroaralkyl, aminoalkyl and mono- and disubstituted aminoalkyl radicals, wherein said substituents are selected from alkyl, aryl, aralkyl, cycloalkyl, cycloalkylalkyl, heteroaryl, heteroaralkyl, heterocycloalkyl, and heterocycloalkylalkyl radicals, or in the case of a disubstituted aminoalkyl radical, said substituents along with the nitrogen atom to which they are attached, form a heterocycloalkyl or a heteroaryl radical. Preferably, R³ represents radicals as defined above which contain no alpha-branching, e.g., as in an isopropyl radical or a t-butyl radical. The preferred radicals are those which contain a -CH₂-moiety between the nitrogen and the remaining portion of the radical. Such preferred groups include, but are not limited to, benzyl, isobutyl, n-butyl, isoamyl, cyclohexylmethyl and the like.

P¹ and P² independently are selected from amine protecting groups, including but not limited to, arylalkyl, substituted arylalkyl, cycloalkenylalkyl and substituted cycloalkenylalkyl, allyl, substituted allyl, acyl, alkoxycarbonyl, aralkoxycarbonyl and silyl. Examples of arylalkyl include, but are not limited to benzyl, ortho-methylbenzyl, trityl and benzhydryl, which can be optionally substituted with halogen, alkyl of C₁-C₈, alkoxy, hydroxy, nitro, alkylene, amino, alkylamino, acylamino and acyl, or their salts, such as phosphonium and ammonium salts. Examples of aryl groups include phenyl, naphthalenyl, indanyl, anthracenyl, durenyl, 9-(9-phenylfluorenyl) and phenanthrenyl, cycloalkenylalkyl or substituted cycloalkylenylalkyl radicals containing cycloalkyls of C₆-C₁₀. Suitable acyl groups include carbobenzoxy, t-butoxycarbonyl, iso-butoxycarbonyl, benzoyl, substituted benzoyl such as 2-methylbenzoyl, 2,6-dimethylbenzoyl 2,4,6-trimethylbenzoyl and 2,4,6-triisopropylbenzoyl, 1-naphthoyl, 2-naphthoyl butyryl, acetyl, tri-fluoroacetyl, tri-chloroacetyl, phthaloyl and the like.

Additionally, the P¹ and/or P² protecting groups can form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, e.g., nitrophthalimidyl. The term silyl refers to a silicon atom optionally substituted by one or more alkyl, aryl and aralkyl groups.

Suitable carbamate protecting groups include, but are not limited to, methyl and ethyl carbamate; 9-fluorenylmethyl carbamate; 9-(2-Sulfo)fluorenylmethyl carbamate; 9-(2,7-dibromo)fluorenylmethyl carbamate; 2,7-di-t-butyl-[9-(10,10-dioxo-10,10,10-tetrahydrothioxanthyl)methyl carbamate; 4-methoxyphenacyl carbamate; 2,2,2-trichloroethyl carbamate; 2-trimethylsilylethyl carbamate; 2-phenylethyl carbamate; 1-(1-adamantyl)-1-methylethyl carbamate; 1,f-dimethyl-2-haloethyl carbamate; 1,1-dimethyl-2,2-dibromoethyl carbamate; 1,1-dimethyl-2,2,2-trichloroethyl carbamate; 1-methyl-1-(4-biphenylyl)-ethyl carbamate; 1-(3,5-di-t-butylphenyl)-1-methylethyl carbamate; 2-(2'-and 4'-pynidyl)ethyl carbamate; 2-(N,N-dicyclohexylcarboxamido)ethyl carbamate; t-butyl carbamate; 1-adamantyl carbamate; vinyl carbamate; allyl carbamate; 1-isopropylallyl carbamate; cinnamyl carbamate; 4-nitrocinnamyl carbamate; 8-quinolyl carbamate; N-hydroxypiperidinyl carbamate; alkyldithio carbamate; benzyl carbamate; p-methoxybenzyl carbamate; p-nitrobenzyl carbamate; p-bromobenzyl carbamate; p-chlorobenzyl carbamate; 2,4-dichlorobenzyl carbamate; 4-methylsulfinylbenzyl carbamate; 9-anthrylmethyl carbamate; diphenylmethyl carbamate; 2-methylthioethyl carbamate; 2-methylsulfonylethyl carbamate; 2-(p-toluenesulfonyl)ethyl carbamate; [2-(1,3-dithianyl)methyl carbamate; 4-methylthiophenyl-2,4-dimethyithiophenyl, 2-phosphonioethyl carbamate; 2-triphenylphosphonioisopropyl carbamate; 1,1-dimethyl-2-cyanoethyl carbamate; m-chloro-p-acyloxybenzyl carbamate; p-(dihydroxyboryl)benzyl carbamate; 5-benziosoxazolylmethyl carbamate; 2-(trifluoromethyl)-6-chromonylmethyl carbamate; m-nitrophenyl carbamate; 3,5-dimethoxybenzyl carbamate; o-nitrobenzyl carbamate; 3,4-dimethoxy-6-nitrobenzyl carbamate; phenyl(o-nitrophenyl)methyl carbamate; phenothiazinyl-(10)-carbonyl derivative; N'-p-toluenesulfonylaminocarbonyl derivative; N'-phenylaminothiocarbonyl derivative t-amyl carbamate; S-benzyl thiocarbamate; p-cyanobenzyl carbamate; cyclobutyl carbamate; cyclohexyl carbamate; cyclopentyl carbamate; cyclopropylmethyl carbamate; p-decyloxybenzyl carbamate; diisopropylmethyl carbamate; 2,2-dimethoxycarbonylvinyl carbamate; o-(N,N-dimethylcarboxamido)benzyl carbamate; 1,1-dimethyl-3-(N,N-dimethylcarboxamido)propyl carbamate; 1,1-dimethylpropynyl carbamate; di(2-pyridyl)methyl carbamate; 2-furanylmethyl carbamate; 2-iodoethyl carbamate; isobornyl carbamate; isobutyl carbamate; isonicotinyl carbamate; p-(p'-methoxyphenylazo)benzyl carbamate; 1-methylcyclobutyl carbamate; 1-methylcyclohexyl carbamate; 1-methyl-1-cyclopropylmethyl carbamate; 1-methyl-1-(3,5-dimethoxyphenyl)ethyl carbamate; 1-methyl-1-(p-phenylazophenyl)ethyl carbamate; and 1-methyl-1-phenylethyl carbamate. T. Greene and P. Wuts ("Protective Groups In Organic Synthesis," 2nd Ed., John Wiley & Sons, Inc. (1991)) describe the preparation and cleavage of such carbamate protecting groups.

Suitable silyl protecting groups include, but are not limited to, trimethyisilyl, triethylsilyl, tri-isopropylsilyl, tert-butyldimethylsilyl, dimethylphenyisilyl, 1,2-bis(dimethylsilyl)benzene, 1,2-bis(dimethyisilyl)ethane and diphenylmethylsilyl. Silylation of the amine functions to provide mono- or bis-disilylamine can provide derivatives of the aminoalcohol, amino acid, amino acid esters and amino acid amide. In the case of amino acids, amino acid esters and amino acid amides, reduction of the carbonyl function provides the required mono- or bis-silyl aminoalcohol. Silylation of the aminoalcohol can lead to the N,N,O-tri-silyl derivative. Removal of the silyl function from the silyl ether function is readily accomplished by treatment with, for example, a metal hydroxide or ammonium flouride reagent, either as a discrete reaction step or in situ during the preparation of the amino aldehyde reagent. Suitable silylating agents are, for example, trimethylsilyl chloride, tert-buty-dimethylsilyl chloride, phenyldimethylsilyl chlorie, diphenylmethylsilyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art or organic chemistry including amino acid/amino acid ester or aminoalcohol chemistry.

Preferably P¹ is selected from aralkyl, substituted aralkyl, alkylcarbonyl, aralkylcarbonyl, arylcarbonyl, alkoxycarbonyl and aralkoxycarbonyl, P² is selected from aralkyl and substituted aralkyl and R¹ is selected from aralkyl and substituted aralkyl. Alternatively, when P¹ is alkoxycarbonyl or aralkoxycarbonyl, P² can be hydrogen. More preferably, P¹ is t-butoxycarbonyl, phenylmethoxycarbonyl or benzyl, P² is hydrogen or benzyl and R¹ is benzyl.

The method comprises the steps of forming a N,N-protected aminoalcohol of formula (II), 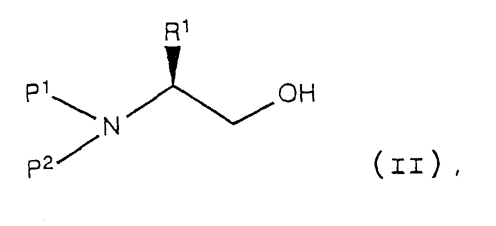 oxidizing said N,N-protected aminoalcohol to a chiral N,N-protected aminoaldehyde of formula (III) 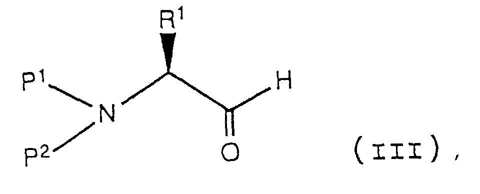 and diastereoselectively forming an aminoepoxide from said aminoaldehyde with an organometallic methylene-adding reagent selected from a halomethyllithium compound in an appropriate solvent and reacting said aminoepoxide with an amine of the formula R³NH₂ wherein R³ is as defined above to form said substituted aminoalcohol.

Protected amino epoxides of the formula 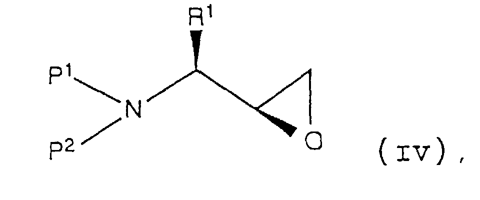 protected amino alpha-hydroxycyanides and acids of the formula 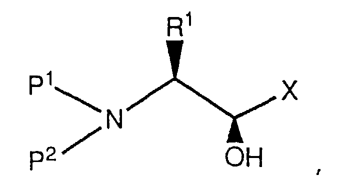 wherein X is -CN, -CH₂NO₂ or -COOH, protected alpha-aminoaldehyde intermediates of the formula 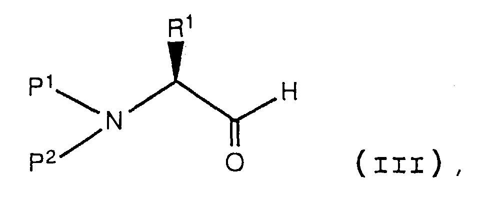 and protected chiral alpha-amino alcohols of the formula 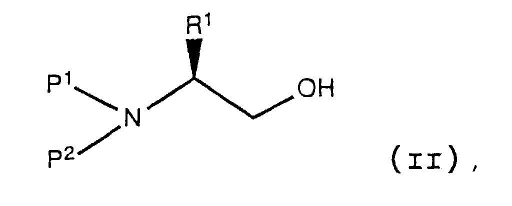 wherein P¹, P² and R¹ are as defined above, are also described herein.

As utilized herein, the term "amino epoxide" alone or in combination; means an amino-substituted alkyl epoxide wherein the amino group can be a primary, or secondary amino group containing substituents selected from hydrogen, and alkyl, aryl, aralkyl, alkenyl, alkoxycarbonyl, aralkoxycarbonyl, cycloalkenyl, silyl, cycloalkylalkenyl radicals and the like and the epoxide can be alpha to the amine. The term "amino aldehyde" alone or in combination, means an amino-substituted alkyl aldehyde wherein the amino group can be a primary, or secondary amino group containing substituents selected from hydrogen, and alkyl, aryl, aralkyl, alkenyl, aralkoxycarbonyl, alkoxycarbonyl, cycloalkenyl, silyl, cycloalkylalkenyl radicals and the like and the aldehyde can be alpha to the amine. The term "alkyl", alone or in combination, means a straight-chain or branched-chain alkyl radical containing from 1 to about 10, preferably from 1 to about 8, carbon atoms. Examples of such radicals include methyl, ethyl, n-propyl, isopropyl, n-butyl, isobutyl, sec-butyl, tert-butyl, pentyl, iso-amyl, hexyl, octyl and the like. The term "alkenyl", alone or in combination, means a straight-chain or branched-chain hydrocarbon radial having one or more double bonds and containing from 2 to about 18 carbon atoms preferably from 2 to about 8 carbon atoms. Examples of suitable alkenyl radicals include ethenyl, propenyl, allyl, 1,4-butadienyl and the like. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy and the like. The term "cycloalkenyl", alone or in combination, means an alkyl radical which contains from about 3 to about 8 carbon atoms and is cyclic and which contains at least one double bond in the ring which is non-aromatic in character. The term "alkynyl", alone or in combination, means a straight-chain hydrocarbon radical having one or more triple bonds and containing from 2 to about 10 carbon atoms. Examples of alkynyl radicals include ethynyl, propynyl, (propargyl), butynyl and the like. The term "cycloalkenylalkyl" means cycloalkenyl radical as defined above which is attached to an alkyl radical, the cyclic portion containing from 3 to about 8, preferably from 3 to about 6, carbon atoms. Examples of such cycloalkenyl radicals include cyclopropenyl, cyclobutenyl, cyclopentenyl, cyclohexenyl, dihydrophenyl and the like. The term "cycloalkyl", alone or in combination, means an alkyl radical which contains from about 3 to about 8 carbon atoms and is cyclic. The term "cycloalkylalkyl" means an alkyl radical as defined above which is substituted by a cycloalkyl radical containing from about 3 to about 8, preferably from about 3 to about 6, carbon atoms. Examples of such cycloalkyl radicals include cyclopropyl, cyclobutyl, cyclopentyl, cyclohexyl and the like. The term "aryl", alone or in combination, means a carbocyclic aromatic system containing one, two or three rings wherein such rings may be attached together in a pendent manner or may be fused. Examples of "aryl" include phenyl or naphthyl radical either of which optionally carries one or more substituents selected from alkyl, alkoxy, halogen, hydroxy, amino, nitro and the like, as well as p-tolyl, 4-methoxyphenyl, 4-(tert-butoxy)phenyl, 4-fluorophenyl, 4-chlorophenyl, 4-hydroxyphenyl, 1-naphthyl, 2-naphthyl, and the like. The term "aralkyl", alone or in combination, means an alkyl radical as defined above in which one hydrogen atom is replaced by an aryl radical as defined above, such as benzyl, 2-phenylethyl and the like. Examples of substituted aralkyl include 3,5-dimethoxybenzyl bromide, 3,4-dimethoxybenzyl bromide, 2,4-dimethoxybenzyl bromide, 3,4,5-trimethoxybenzyl bromide, 4-nitrobenzyl iodide, 2,6-dichlorobenzyl bromide, 1,4-bis(chloromethyl)benzene, 1,2-bis(bromomethyl)benzene, 1,3-bis(chloromethyl)-benzene, 4-chlorobenzyl chloride, 3-chlorobenzyl. chloride, 1,2-bis(chloromethyl)benzene, 6-chloropiperonyl chloride, 2-chlorobenzyl chloride, 4-chloro-2-nitrobenzyl chloride, 2-chloro-6-fluorobenzyl chloride, 1,2-bis(chloromethyl)-4,5-dimethylbenzene, 3,6-bis(chloromethyl)durene, 9,10-bis(chloromethyl)anthracene, 2,5-bis(chloromethyl)-p-xylene, 2,5-bis(chloromethyl)-1,4-dimethoxybenzene, 2,4-bis(chloromethyl)anisole, 4,6-(dichloromethyl)-m-xylene, 2,4-bis(chloromethyl)mesitylene, 4-(bromomethyl)-3,5-dichlorobenzophenone, n-(alpha-chloro-o-tolyl)benzylamine hydrochloride, 3-(chloromethyl)benzoyl chloride, 2-chloro-4-chloromethyltoluene, 3,4-dichlorobenzyl bromide, 6-chloro-8-chloromethylbenzo-1,3-dioxan, 4-(2,6-dichlorobenzylsulphonyl)benzylbromide, 5-(4-chloromethylphenyl)-3-(4-chlorophenyl)-1,2,4-oxadiazole, 5-(3-chloromethylphenyl)-3-(4-chlorophenyl)-1,2,4-oxadiazole, 4-(chloromethyl)benzoyl chloride, di(chloromethyl)toluene, 4-chloro-3-nitrobenzyl chloride, 1-(dimethylchlorosilyl)-2-(p,m-chloromethylphenyl)ethane, 1-(dimethylchlorosilyl)-2-(p,m-chloromethylphenyl)ethane, 3-chloro-4-methoxybenzyl chloride, 2,6-bis(chloromethyl)-4-methylphenol, 2,6-bis(chloromethyl)-p-tolyl acetate, 4-bromobenzyl bromide, p-bromobenzoyl bromide, alpha alpha'-dibromo-m-xylene, 3-bromobenzyl bromide, 2-bromobenzyl bromide, 1,8-bis(bromomethyl)naphthalene, o-xylylene dibromide, p-xylylene dibromide, 2,2'-bis(bromomethyl)-1,1'-biphenyl, alpha,alpha'-dibromo-2,5-dimethoxy-p-xylene, benzyl chloride, benzyl bromide, 4,5-bis(bromomethyl)phenanthrene, 3-(bromomethyl)benzyltriphenylphosphonium bromide, 4-(bromomethyl)benzyltriphenylphosphonium bromide, 2-(bromomethyl)benzyltriphenylphosphonium bromide, 1-(2-bromoethyl)-2-(bromomethyl)-4-nitrobenzene, 2-bromo-5-fluorobenzylbromide, 2,6-bis(bromomethyl) fluorobenzene, o-bromomethylbenzoyl bromide, p-bromomethyl benzoyl bromide, 1-bromo-2-(bromomethyl)naphthalene, 2-bromo-5-methoxybenzyl bromide, 2,4-dichlorobenzyl chloride, 3,4-dichlorobenzyl chloride, 2,6-dichlorobenzyl chloride, 2,3-dichlorobenzyl chloride, 2,5-dichlorobenzyl chloride, methyldichlorosilyl(chloromethylphenyl)ethane, methyldichlorosilyl(chloromethylphenyl)ethane, methyldichlorosilyl(chloromethylphenyl)ethane, 3,5-dichlorobenzyl chloride, 3,5-dibromo-2-hydroxybenzyl bromide, 3,5-dibromobenzyl bromide, p-(chloromethyl)phenyltrichlorosilane, 1-trichlorosilyl-2-(p,m-chloromethylphenyl)ethane, 1-trichlorosilyl-2-(p,m-chloromethylphenyl)ethane, 1,2,4,5-tetrakis(bromomethyl)benzene. The term aralkoxycarbonyl means an aralkoxyl group attached to a carbonyl. Carbobenzoxy is an example of aralkoxycarbonyl. The term "heterocyclic ring system" means a saturated or partially unsaturated monocyclic, bicyclic or tricyclic heterocycle which contains one or more hetero atoms as ring atoms, selected from nitrogen, oxygen, silicon and sulphur, which is optionally substituted on one or more carbon atoms by halogen, alkyl, alkoxy, oxo, and the like, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group or the like is an aromatic monocyclic, bicyclic, or tricyclic heterocycle which contains the hetero atoms and is optionally substituted as defined above with respect to the definition of aryl. Examples of such heterocyclic groups are pyrrolidinyl, piperidinyl, piperazinyl, morpholinyl, thiamorpholinyl, pyrrolyl, phthalimide, succinimide, maleimide, and the like. Also included are heterocycles containing two silicon atoms simultaneously attached to the nitrogen and joined by carbon atoms. The term "alkylamino" alone or in combination, means an amino-substituted alkyl group wherein the amino group can be a primary, or secondary amino group containing substituents selected from hydrogen, and alkyl, aryl, aralkyl, cycloalkyl, cycloalkylalkyl radicals and the like. The term "halogen" means fluorine, chlorine, bromine or iodine. The term dihaloalkyl means two halogen atoms, the same or different, substituted on the same carbon atom. The term "oxidizing agent" includes a single agent or a mixture of oxidizing reagents. Examples of mixtures of oxidizing reagents include sulfur trioxide-pyridine/dimethylsulfoxide, oxalyl chloride/dimethyl sulfoxide, acetyl chloride/dimethyl sulfoxide, acetyl anhydride/dimethyl sulfoxide, trifluoroacetyl chloride/dimethyl sulfoxide, toluenesulfonyl bromide/dimethyl sulfoxide, phosphorous pentachloride/dimethyl sulfoxide and isobutylchloroformate/dimethyl sulfoxide.

A general Scheme for the preparation of amino epoxides, useful as intermediates in the synthesis of HIV protease inhibitors is shown in Scheme 1 below.

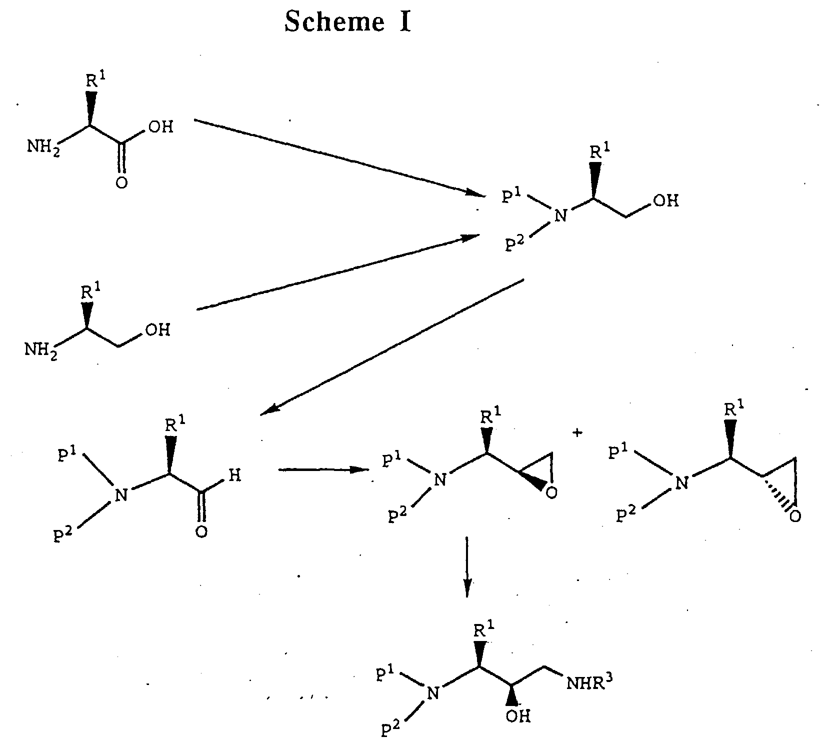

The economical and safe large scale method of preparation of protease inhibitors of the present invention can alternatively utilize amino acids or amino alcohols to form N,N-protected alpha aminoalcohol of the formula 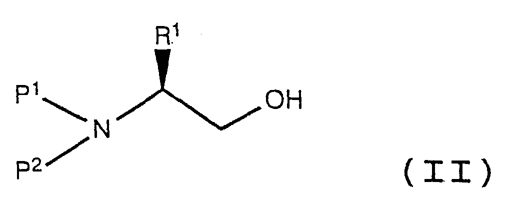 wherein P¹, P² and R¹ are described above.

Whether the compounds of Formula II are formed from amino acids or aminoalcohols, such compounds have the amine protected with groups P¹ and P² as previously identified. The nitrogen atom can be alkylated such as by the addition of suitable alkylating agents in an appropriate solvent in the presence of base.

Alternate bases used in alkylation include sodium hydroxide, sodium bicarbonate, potassium hydroxide, lithium hydroxide, potassium carbonate, sodium carbonate, cesium hydroxide, magnesium hydroxide, calcium hydroxide or calcium oxide, or tertiary amine bases such as triethyl amine, diisopropylethylamine, N-methylpiperidine, pyridine, dimethylaminopyridine and azabicyclononane. Reactions can be homogenous or heterogenous. Suitable solvents are water and protic solvents or solvents miscible with water, such as methanol, ethanol, isopropyl alcohol, tetrahydrofuran and the like, with or without added water. Dipolar aprotic solvents may also be used with or without added protic solvents including water. Examples of dipolar aprotic solvents include acetonitrile, dimethylformamide, dimethyl acetamide, acetamide, tetramethyl urea and its cyclic analog, dimethylsulfoxide, N-methylpyrrolidone, sulfolane, nitromethane and the like. Reaction temperature can range between about -20° to 100°C with the preferred temperature of about 25-85°C. The reaction may be carried out under an inert atmosphere such as nitrogen or argon, or normal or dry air, under atmospheric pressure or in a sealed reaction vessel under positive pressure. The most preferred alkylating agents are benzyl bromide or benzyl chloride or monosubstituted aralkyl halides or polysubstituted aralkyl halides. Sulfate or sulfonate esters are also suitable reagents to provide the corresponding benzyl analogs and they can be preformed from the corresponding benzyl alcohol or formed in situ by methods well known to those skilled in the art. Trityl, benzhydryl, substituted trityl and substituted benzhydryl groups, independently, are also effective amine protecting groups [P¹,P²] as are allyl and substituted allyl groups. Their halide derivatives can also be prepared from the corresponding alcohols by methods well known to those skilled in the art such as treatment with thionyl chloride or bromide or with phosphorus tri- or pentachloride, bromide or iodide or the corresponding phosphoryl trihalide. Examples of groups that can be substituted on the aryl ring include alkyl, alkoxy, hydroxy, nitro, halo and alkylene, amino, mono- and dialkyl amino and acyl amino, acyl and water solubilizing groups such as phosphonium salts and ammonium salts. The aryl ring can be derived from, for example, benzene, napthelene, indane, anthracene, 9-(9-phenyl fluorenyl, durene, phenanthrene and the like. In addition, 1,2-bis (substituted alkylene) aryl halides or sulfonate esters can be used to form a nitrogen containing aryl or non-aromatic heterocyclic derivative [with P¹ and P²] or bis-heterocycles. Cycloalkylenealkyl or substituted cyloalkylene. radicals containing 6-10 carbon atoms and alkylene radicals constitute additional acceptable class of substituents on nitrogen prepared as outlined above including, for example, cyclohexylenemethylene.

Compounds of Formula II can also be prepared by reductive alkylation by, for example, compounds and intermediates formed from the addition of an aldehyde with the amine and a reducing agent, reduction of a Schiff Base, carbinolamine or enamine or reduction of an acylated amine derivative. Reducing agents include metals [platinum, palladium, palladium hydroxide, palladium on carbon, platinum oxide, rhodium and the like] with hydrogen gas or hydrogen transfer molecules such as cyclohexene or cyclohexadiene or hydride agents such as lithium aluminumhydride, sodium borohydride, lithium borohydride, sodium cyanoborohydride, diisobutylaluminum hydride or lithium tri-tert-butoxyaluminum hydride.

Additives such as sodium or potassium bromide, sodium or potassium iodide can catalyze or accelerate the rate of amine alkylation, especially when benzyl chloride was used as the nitrogen alkylating agent.

Phase transfer catalysis wherein the amine to be protected and the nitrogen alkylating agent are reacted with base in a solvent mixture in the presence of a phase transfer reagent, catalyst or promoter. The mixture can consist of, for example, toluene, benzene, ethylene dichloride, cyclohexane, methylene chloride or the like with water or a aqueous solution of an organic water miscible solvent such as THF. Examples of phase transfer catalysts or reagents include tetrabutylammonium chloride or iodide or bromide, tetrabutylammonium hydroxide, tri-butyloctylammonium chloride, dodecyltrihexylammonium hydroxide, methyltrihexylammonium chloride and the like.

A preferred method of forming substituted amines involves the aqueous addition of about 3 moles of organic halide to the amino acid or about 2 moles to the aminoalcohol. In a more preferred method of forming a protected amino alcohol, about 2 moles of benzylhalide in a basic aqueous solution is utilized. In an even more preferred method, the alkylation occurs at 50°C to 80°C with potassium carbonate in water, ethanol/water or denatured ethanol/water. In a more preferred method of forming a protected amino acid ester, about 3 moles of benzylhalide is added to a solution containing the amino acid.

The protected amino acid ester is additionally reduced to the protected amino alcohol in an organic solvent. Preferred reducing agents include lithium aluminiumhydride, lithium borohydride, sodium borohydride, borane, lithium tri-tert-butoxyaluminum hydride, borane·THF complex. Most preferably, the reducing agent is diisobutylaluminum hydride (DiBAL-H) in toluene. These reduction conditions provide an alternative to a lithium aluminum hydride reduction.

Purification by chromatography is possible. In the preferred purification method the alpha amino alcohol can be purified by an acid quench of the reaction, such as with hydrochloric acid, and the resulting salt can be filtered off as a solid and the amino alcohol can be liberated such as by acid/base extraction.

The protected alpha amino alcohol is oxidized to form a chiral amino aldehyde of the formula 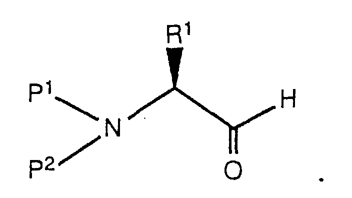 Acceptable oxidizing reagents include, for example, sulfur trioxide-pyridine complex and DMSO, oxalyl chloride and DMSO, acetyl chloride or anhydride and DMSO, trifluoroacetyl chloride or anhydride and DMSO, methanesulfonyl chloride and DMSO or tetrahydrothiaphene-S-oxide, toluenesulfonyl bromide and DMSO, trifluoromethanesulfonyl anhydride (triflic anhydride) and DMSO, phosphorus pentachloride and DMSO, dimethylphosphoryl chloride, and DMSO and isobutylchloroformate and DMSO. The oxidation conditions reported by Reetz et al [Angew Chem., 99, p. 1186, (1987)], Angew Chem. Int. Ed. Engl., 26, p. 1141, 1987) employed oxalyl chloride and DMSO at -78°C.

The preferred oxidation method described in this invention is sulfur trioxide pyridine complex, triethylamine and DMSO at room temperature. This system provides excellent yields of the desired chiral protected amino aldehyde usable without the need for purification i.e., the need to purify kilograms of intermediates by chromatography is eliminated and large scale operations are made less hazardous. Reaction at room temperature also eliminated the need for the use of low temperature reactor which makes the process more suitable for commercial production.

The reaction may be carried out under an inert atmosphere such as nitrogen or argon, or normal or dry air, under atmospheric pressure or in a sealed reaction vessel under positive pressure. Preferred is a nitrogen atmosphere. Alternative amine bases include, for example, tri-butyl amine, tri-isopropyl amine, N-methylpiperidine, N-methyl morpholine, azabicyclononane, diisopropylethylamine, 2,2,6,6-tetramethylpiperidine, N,N-dimethylaminopyridine, or mixtures of these bases. Triethylamine is a preferred base. Alternatives to pure DMSO as solvent include mixtures of DMSO with non-protic or halogenated solvents such as tetrahydrofuran, ethyl acetate, toluene, xylene, dichloromethane, ethylene dichloride and the like. Dipolar aprotic co-solvents include acetonitrile, dimethylformamide, dimethylacetamide, acetamide, tetramethyl urea and its cyclic analog, N-methylpyrrolidone, sulfolane and the like. Rather than N,N-dibenzylphenylalaninol as the aldehyde precursor, the phenylalaninol derivatives discussed above can be used to provide the corresponding N-monosubstituted [either P¹ or P² = H] or N,N-disubstituted aldehyde.

In addition, hydride reduction of an amide or ester derivative of the corresponding alkyl, benzyl or cycloalkenyl nitrogen protected phenylalanine, substituted phenylalanine or cycloalkyl analog of phenyalanine derivative can be carried out to provide a compound of Formula III. Hydride transfer is an additional method of aldehyde synthesis under conditions where aldehyde condensations are avoided, cf, Oppenauer Oxidation.

The aldehydes of this process can also be prepared by methods of reducing protected phenylalanine and phenylalanine analogs or their amide or ester derivatives by, e.g., sodium amalgam with HCl in ethanol or lithium or sodium or potassium or calcium in ammonia. The reaction temperature may be from about -20°C to about 45°C, and preferably from abut 5°C to about 25°C. Two additional methods of obtaining the nitrogen protected aldehyde include oxidation of the corresponding alcohol with bleach in the presence of a catalytic amount of 2,2,6,6-tetramethyl-1-pyridyloxy free radical. In a second method, oxidation of the alcohol to the aldehyde is accomplished by a catalytic amount of tetrapropylammonium perruthenate in the presence of N-methylmorpholine-N-oxide.

Alternatively, an acid chloride derivative of a protected phenylalanine or phenylalanine derivative as disclosed above can be reduced with hydrogen and a catalyst such as Pd on barium carbonate or barium sulphate, with or without an additional catalyst moderating agent such as sulfur or a thiol (Rosenmund Reduction).

An important aspect of the present invention is a reaction involving the addition of chloromethylithium or bromomethyllithium to the α-amino aldehyde. Although addition of chloromethyllithium or bromomethylithium to aldehydes is known, the addition of such species to racemic or chiral amino aldehydes to form aminoepoxides of the formula 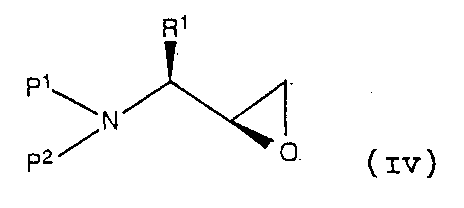 is novel. The addition of chloromethylithium or bromomethylithium to a chiral amino aldehyde is highly diastereoselective. Preferably, the chloromethyllithium or bromomethylithium is generated in-situ from the reaction of the dihalomethane and n-butyllithium. Acceptable methyleneating halomethanes include chloroiodomethane, bromochloromethane, dibromomethane, diiodomethane, bromofluoromethane and the like. The sulfonate ester of the addition product of, for example, hydrogen bromide to formaldehyde is also a methyleneating agent. Tetrahydrofuran is the preferred solvent, however alternative solvents such as toluene, dimethoxyethane, ethylene dichloride, methylene chloride can be used as pure solvents or as a mixture. Dipolar aprotic solvents such as acetonitrile, DMF, N-methylpyrrolidone are useful as solvents or as part of a solvent mixture. The reaction can be carried out under an inert atmosphere such as nitrogen or argon. For n-butyl lithium can be substituted other organometalic reagents reagents such as methyllithium, tert-butyl lithium, sec-butyl lithium, phenyllithium, phenyl sodium and the like. The reaction can be carried out at temperatures of between about -80°C to 0°C but preferably between about -80°C to -20°C. The most preferred reaction temperatures are between -40°C to -15°C. Reagents can be added singly but multiple additions are preferred in certain conditions. The preferred pressure of the reaction is atmospheric however a positive pressure is valuable under certain conditions such as a high humidity environment.

Alternative methods of conversion to the epoxides of this invention include substitution of other charged methylenation precurser species followed by their treatment with base to form the analogous anion. Examples of these species include trimethylsulfoxonium tosylate or triflate, tetramethylammonium halide, methyldiphenylsulfoxonium halide wherein halide is chloride, bromide or iodide.

The conversion of the aldehydes of this invention into their epoxide derivative can also be carried out in multiple steps. For example, the addition of the anion of thioanisole prepared from, for example, a butyl or aryl lithium reagent, to the protected aminoaldehyde, oxidation of the resulting protected aminosulfide alcohol with well known oxidizing agents such as hydrogen peroxide, tert-butyl hypochlorite, bleach or sodium periodate to give a sulfoxide. Alkylation of the sulfoxide with, for example, methyl iodide or bromide, methyl tosylate, methyl mesylate, methyl triflate, ethyl bromide, isopropyl bromide, benzyl chloride or the like, in the presence of an organic or inorganic base Alternatively, the protected aminosulfide alcohol can be alkylated with, for example, the alkylating agents above, to provide a sulfonium salts that are subsequently converted into the subject epoxides with tert-amine or mineral bases.

The desired epoxides form, using most preferred conditions, diastereoselectively in ratio amounts of at least about an 85:15 ratio (S:R). The product can be purified by chromatography to give the diastereomerically and enantiomerically pure product but it is more conveniently used directly without purification to prepare HIV protease inhibitors.
The epoxide is then reacted, in a suitable solvent system, with an equal amount, or preferably an excess of, with R³NH₂ to form the amino alcohol of Formula I 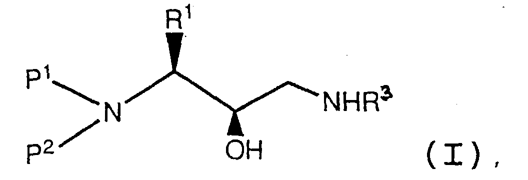 wherein R³ is as defined above.

The reaction can be conducted over a wide range of temperatures, e.g., from about 10°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. In some cases, R³NH₂ can be used as the solvent, such as isobutylamine.

Alternatively, the amine of Formula I can be prepared by reduction of the protected amino acid of formula 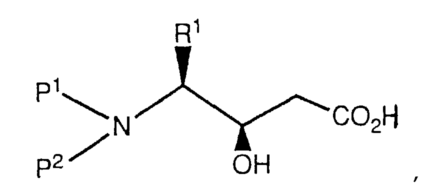 (commercially available from Nippon Kayaku, Japan) to the corresponding alcohol of formula 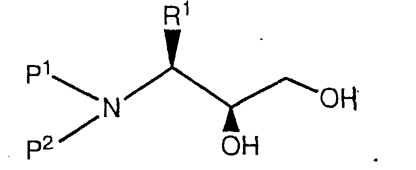 The reduction can be accomplished using a variety of reducing reagents and conditions. A preferred reducing reagent is diborane·tetrahydrofuran. The alcohol is then converted into a leaving group (L') by tosylation, mesylation or conversion into a halo group, such as chloro or bromo: 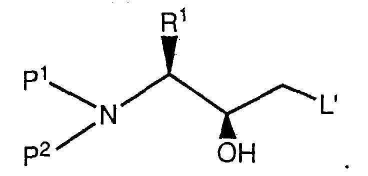 Finally, the leaving group (L') is reacted with R³NH₂ as described above to form amino alcohol of Formula I. Alternatively, base treatment of the alcohol can result in the formation of the amino epoxide of Formula IV.

The above preparation of amino alcohol of Formula I is applicable to mixtures of optical isomers as well as resolved compounds. If a particular optical isomer is desired, it can be selected by the choice of starting material, e.g., L-phenylalanine, D-phenylalanine, L-phenylalaninol, D-phenylalaninol, D-hexahydrophenylalaninol and the like, or resolution can occur at intermediate or final steps. Chiral auxiliaries such as one or two equivalents of camphor sulfonic acid, citric acid, camphoric acid, 2-methoxyphenylacetic acid and the like can be used to form salts, esters or amides of the compounds of this invention. These compounds or derivatives can be crystallized or separated chromatographically using either a chiral or achiral column as is well known to those skilled in the art.

A further advantage of the present process is that materials can be carried through the above steps without purification of the intermedate products. However, if purification is desired, the intermediates disclosed can be prepared and stored in a pure state.

The practical and efficient synthesis described here has been successfully scaled up to prepare large quantity of intermediates for the preparation of HIV protease inhibitors. It offers several advantages for multikilogram preparations: (1) it does not require the use of hazardous reagents such as diazomethane, (2) it requires no purification by chromatography, (3) it is short and efficient, (4) it utilizes inexpensive and readily available commercial reagents, (5) it produces enantiomerically pure alpha amino epoxides. In particular, the process of the invention produces enantiomerically-pure epoxide as required for the preparation of enantiomerically-pure intermediate for further synthesis of HIV protease inhibitors.

The amino epoxides were prepared utilizing the following procedure as disclosed in Scheme II below.

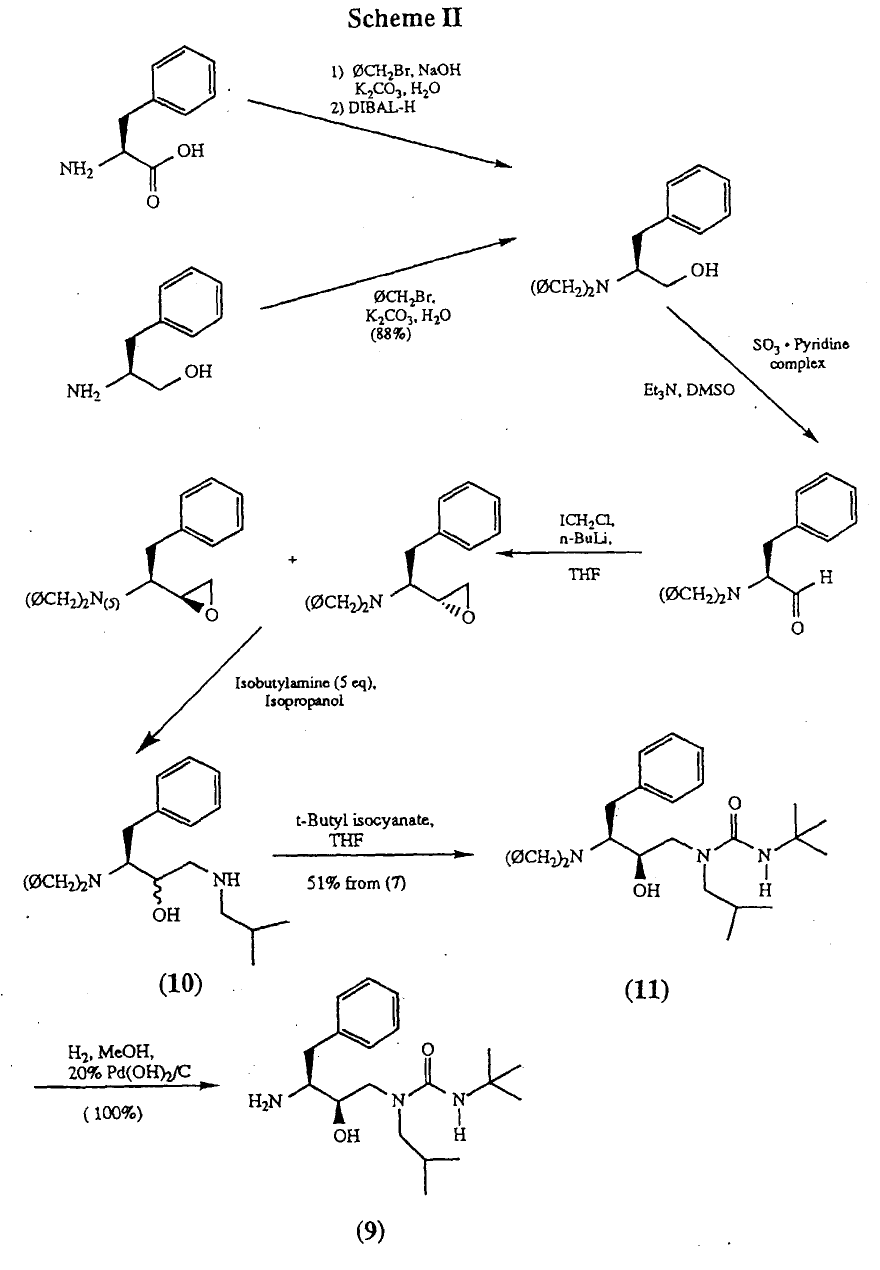

In Scheme II, there is shown a synthesis for the epoxide, chiral N, N,α-S-tris(phenylmethyl)-2S-oxiranemethan-amine. The synthesis starts from L-phenylalanine. The aldehyde is prepared in three steps from L-phenylalanine or phenylalinol. L-Phenylalanine is converted to the N,N-dibenzylamino acid benzyl ester using benzyl bromide under aqueous conditions. The reduction of benzyl ester is carried out using diisobutylaluminum hydride (DIBAL-H) in toluene. Instead of purification by chromatography, the product iss purified by an acid (hydrochloric acid) quench of the reaction, the hydrochloride salt is filtered off as a white solid and then liberated by an acid/base extraction. After one recrystallization, chemically and optically pure alcohol is obtained. Alternately, and preferably, the alcohol can be obtained in one step in 88% yield by the benzylation of L-phenylalaninol using benzylbromide under aqueous conditions. The oxidation of alcohol to aldehyde is also modified to allow for more convenient operation during scaleup. Instead of the standard Swern procedures using oxalyl chloride and DMSO in methylene chloride at low temperatures (very exothermic reaction), sulfur trioxide-pyridine/DMSO was employed (Parikh, J., Doering, W., J. Am. Chem. Soc., 89, p. 5505, 1967) which can be conveniently performed at room temperature to give excellent yields of the desired aldehyde with high chemical and enantiomer purity which does not require purification.

An important reaction involves the addition of chloromethylithium or bromomethylithium to the aldehyde. Although addition of chloromethyllithium or bromomethylithium to aldehydes has been reported previously, the addition of such species to chiral α-amino aldehydes to form chiral-aminoepoxides is believed to be novel. Now, chloromethyllithium or bromomethylithium is generated in-situ from chloroiodomethane (or bromochloromethane) or dibromomethane and n-butyllithium at a temperature in a range from about -78°C to about -10°C in THF in the presence of aldehyde. The desired chlorohydrin or bromohydrin is formed as evidenced by TLC analyses. After warming to room temperature, the desired epoxide is formed diastereoselectively in a 85:15 ratio (S:R). The product can be purified by chromatography to give the diastereomerically pure product as a colorless oil but it is more conveniently used directly without purification.

Scheme III illustrates the preparation of the aminopropylurea (9) utilizing mixed protected amine of phenylalaninol, where BOC is t-butoxycarbonyl and Bn is benzyl.

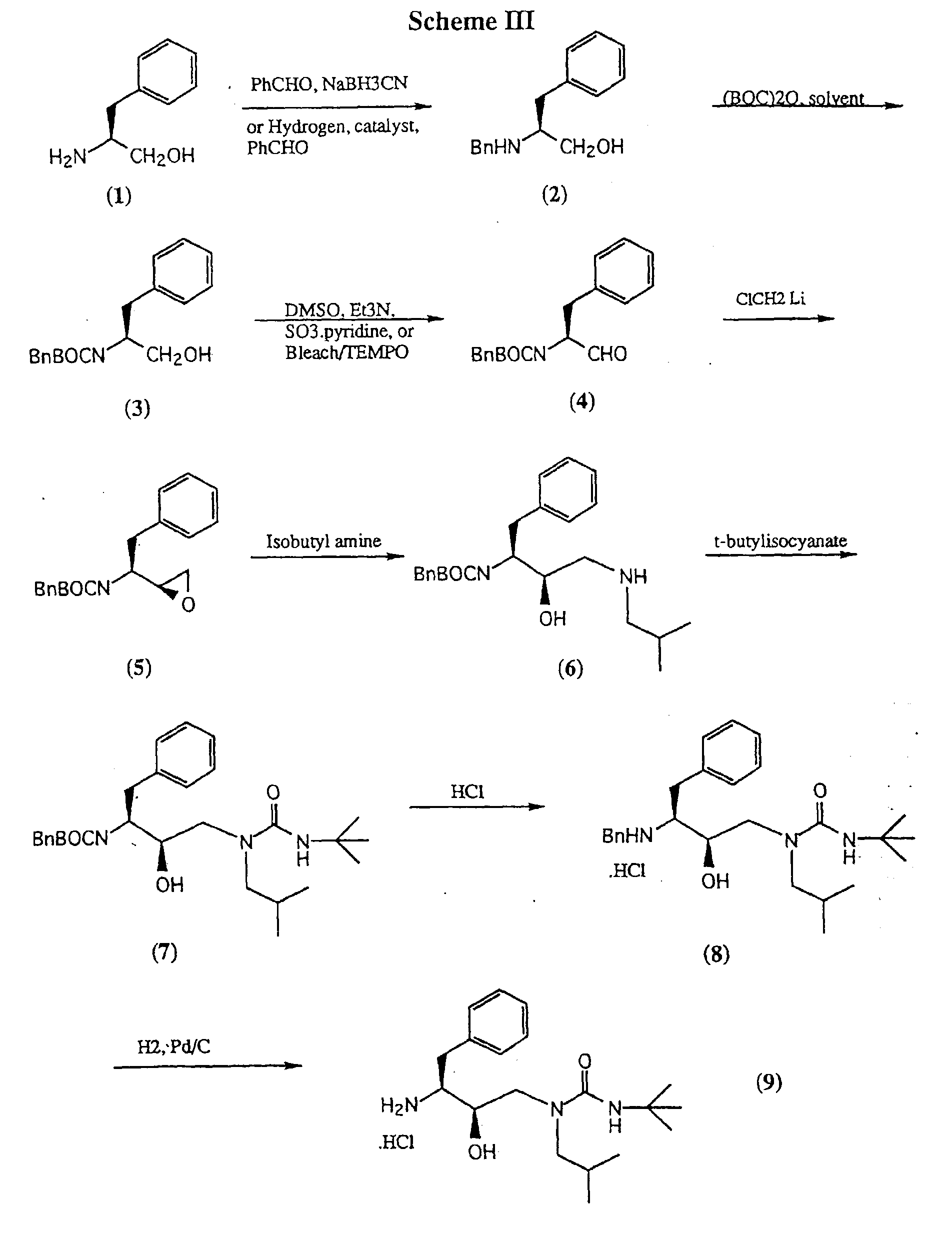

Scheme IV illustrates an alternative preparation of the amino epoxide (5) utilizing a sulfur ylide.

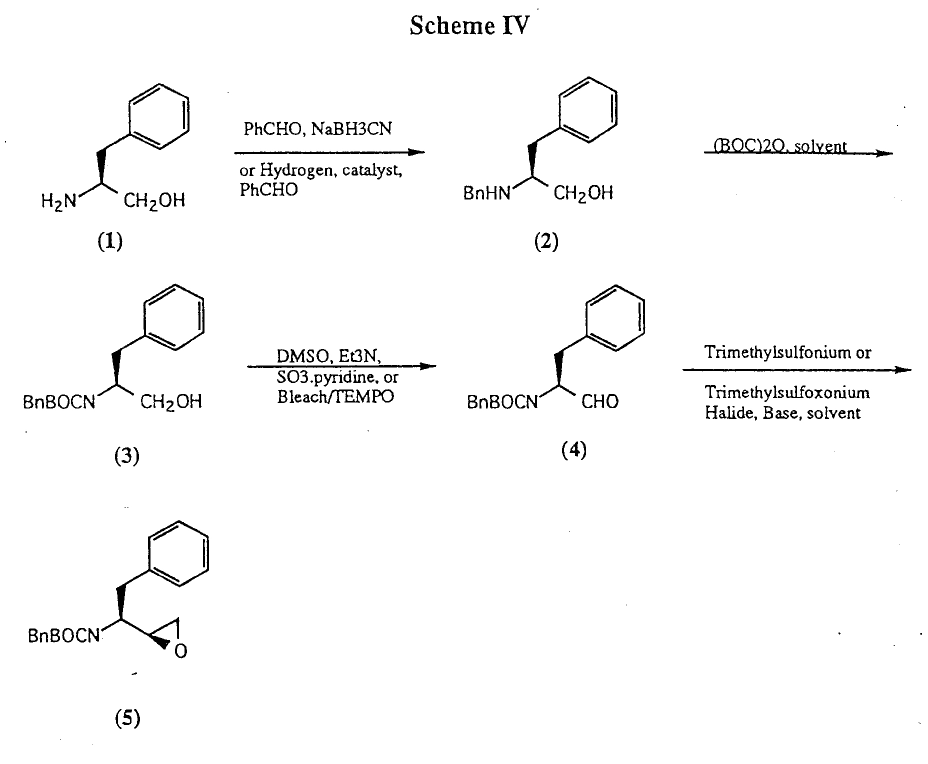

The aminourea (9) was also prepared utilizing the procedure as disclosed in Scheme V below. 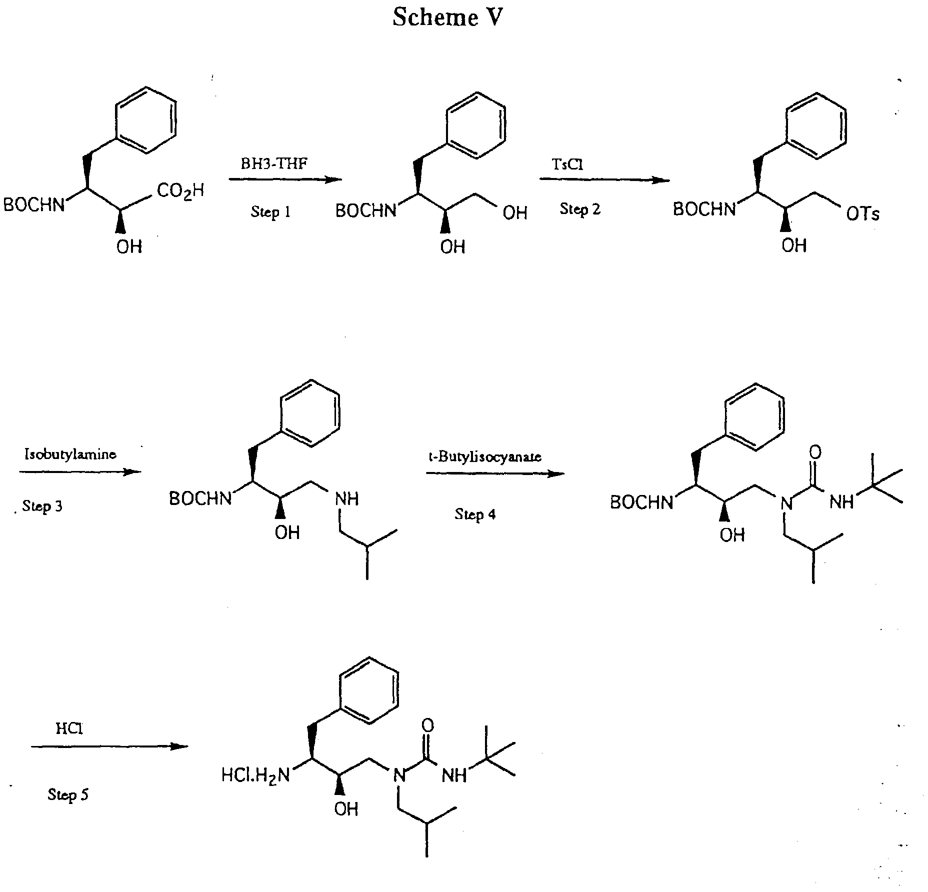 The procedure in Scheme V required only one protecting group, BOC, for the amine of the hydroxyamino acid. This procedure has the advantage of having the desired stereochemistry of the benzyl and hydroxy groups established in the starting material. Thus the chirality does not need to be introduced with the resulting loss of material due to preparation of diastereomers.

### EXAMPLE 1

### β-2-[Bis(phenylmethyl)amino]benzenepropanol

### METHOD 1:

### Step 1: Benzylation of L-Phenylalanine

A solution of L-phenylalanine (50.0 g, 0.302 mol), sodium hydroxide (24.2 g, 0.605 mol) and potassium carbonate (83.6 g, 0.605 mol) in water (500 mL) was heated to 97°C. Benzyl bromide (108.5 mL, 0.605 mol) was then slowly added (addition time - 25 min). The mixture was stirred at 97°C for 30 minutes under a nitrogen atmosphere. The solution was cooled to room temperature and extracted with toluene (2 x 250 mL). The combined organic layers were washed with water and brine, dried over magnesium sulfate, filtered and concentrated to an oil. The identity of the product was confirmed as follows. Analytical TLC (10% ethyl acetate/hexane, silica gel) showed major component at Rf value = 0.32 to be the desired tribenzylated compound, N,N-bis(phenylmethyl)-L-phenylalanine phenylmethyl ester. This compound can be purified by column chromatography (silica gel, 15% ethyl acetate/hexanes). Usually the product is pure enough to be used directly in the next step without further purification. ¹H NMR spectrum was in agreement with published literature. ¹H NMR (CDCL₃) ∂, 3.00 and 3.14 (ABX-system, 2H, J_{AB}=14.1 Hz, J_{AX}=7.3 Hz and J_{BX}= 5.9 Hz), 3.54 and 3.92 (AB-System , 4 H, J_{AB}=13.9 Hz), 3.71 (t, 1H, J=7.6 Hz), 5.11 and 5.23 (AB-System, 2H, J_{AB}=12.3 Hz), and 7.18 (m, 20 H). EIMS: m/z 434 (M-1).

### Step 2: βS-2-[Bis(phenylmethyl)amino]benzenepropanol from the DIBAL Reduction of N,N-bis(phenylmethyl)-L-Phenylalanine phenylmethyl ester

The benzylated phenylalanine phenylmethyl ester (0.302 mol) from the previous reaction was dissolved in toluene (750 mL) and cooled to -55°C. A 1.5 M solution of DIBAL in toluene (443.9 mL, 0.666 mol) was added at a rate to maintain the temperature between -55 to -50°C (addition time - 1 hr). The mixture was stirred for 20 minutes under a nitrogen atmosphere and then quenched at -55°C by the slow addition of methanol (37 ml). The cold solution was then poured into cold (5°C) 1.5 N HCl solution (1.8 L). The precipitated solid (approx. 138 g) was filtered off and washed with toluene. The solid material was suspended in a mixture of toluene (400 mL) and water (100 ml). The mixture was cooled to 5°C and treated with 2.5 N NaOH (186 mL) and then stirred at room temperature until solid dissolved. The toluene layer was separated from the aqueous phase and washed with water and brine, dried over magnesium sulfate, filtered and concentrated to a volume of 75 mL (89 g). Ethyl acetate (25 mL) and hexane (25 mL) were added to the residue upon which the desired alcohol product began to crystallize. After 30 min, an additional 50 mL hexane were added to promote further crystallization. The solid was filtered off and washed with 50 mL hexane to give 34.9 g of first crop product. A second crop of product (5.6 g) was isolated by refiltering the mother liquor. The two crops were combined and recrystallized from ethyl acetate (20 mL) and hexane (30 mL) to give 40 g of βS-2-[Bis(phenyl-methyl)amino]benzenepropanol, 40% yield from L-phenylalanine. An additional 7 g (7%) of product can be obtained, from recrystallyzation of the concentrated mother liquor. TLC of product Rf = 0.23 (10% ethyl acetate/hexane, silica gel);¹H NMR (CDCl₃) ∂ 2.44 (m, 1H,), 3.09 (m, 2H), 3.33 (m, 1H), 3.48 and 3.92 (AB-System, 4H, J_{AB}= 13.3 Hz), 3.52 (m, 1H) and 7.23 (m, 15H); [α]_{D}25 +42.4 (c 1.45, CH₂Cl₂); DSC 77.67°C; Anal. Calcd.for C₂₃H₂₅ON: C, 83.34; H, 7.60; N, 4.23. Found: C, 83.43; H, 7.59; N, 4.22. HPLC on chiral stationary phase: Cyclobond I SP column (250 x 4.6 mm I.D.), mobile phase: methanol/triethyl ammonium acetate buffer pH 4.2 (58:42, v/v), flow-rate of 0.5 ml/min, detection with detector at 230nm and a temperature of 0°C. Retention time: 11.25 min., retention time of the desired product enantiomer: 12.5 min.

### METHOD 2:

### Preparation of βS-2-[Bis(phenylmethyl)amino]benzene-propanol from the N,N-Dibenzylation of L-Phenylalaninol:

L-phenylalaninol (176.6 g, 1.168 mol) was added to a stirred solution of potassium carbonate (484.6 g, 3.506 mol) in 710 mL of water. The mixture was heated to 65°C under a nitrogen atmosphere. A solution of benzyl bromide (400 g, 2.339 mol) in 3A ethanol (305 mL) was added at a rate that maintained the temperature between 60-68°C. The biphasic solution was stirred at 65°C for 55 min and then allowed to cool to 10°C with vigorous stirring. The oily product solidified into small granules. The product was diluted with 2.0 L of tap water and stirred for 5 minutes to dissolve the inorganic by products. The product was isolated by filtration under reduced pressure and washed with water until the pH is 7. The crude product obtained was air dried overnite to give a semi-dry solid (407 g) which was recrystallized from 1.1 L of ethyl acetate/heptane (1:10 by volume). The product was isolated by filtration (at -8°C ), washed with 1.6 L of cold (-10°C ) ethyl acetate/heptane (1:10 by volume) and air-dried to give 339 g (88% yield) of βS-2-[Bis(phenylmethyl)amino]benzene-propanol, mp 71.5-73.0°C. More product can be obtained from the mother liquor if necessary. The other analytical characterization was identical to compound prepared as described in Method 1.

### EXAMPLE 2

### αS-[Bis(phenylmethyl)amino]benzenepropanaldehyde

### METHOD 1:

βS-2-[Bis(phenylmethyl)amino]benzene-propanol (200 g, 0.604 mol) was dissolved in triethylamine (300 mL, 2.15 mol). The mixture was cooled to 12°C and a solution of sulfur trioxide/pyridine complex (380 g, 2.39 mol) in DMSO (1.6 L) was added at a rate to maintain the temperature between 8-17°C (addition time - 1.0 h). The solution was stirred at ambient temperature under a nitrogen atmosphere for 1.5 hour at which time the reaction was complete by TLC analysis (33% ethyl acetate/hexane, silica gel). The reaction mixture was cooled with ice water and quenced with 1.6 L of cold water (10-15°C) over 45 minutes. The resultant solution was extracted with ethyl acetate (2.0 L), washed with 5% citric acid (2.0 L), and brine (2.2 L), dried over MgSO₄ (280 g) and filtered. The solvent was removed on a rotary evaporator at 35-40°C and then dried under vaccuum to give 198.8 g of αS-[Bis-(phenylmethyl)amino]benzenepropanaldehyde as a pale yellow oil (99.9%). The crude product obtained was pure enough to be used directly in the next step without purification. The analytical data of the compound were consistent with the published literature. [α]_{D}25 = -92.9 ° (c 1.87, CH₂Cl₂); ¹H NMR (400 MHz, CDCl₃) ∂, 2.94 and 3.15 (ABX-System, 2H, J_{AB}= 13.9 Hz, J_{AX}= 7.3 Hz and J_{BX} = 6.2 Hz), 3.56 (t, 1H, 7.1 Hz), 3.69 and 3.82 (AB-System, 4H, J_{AB}= 13.7 Hz), 7.25 (m, 15 H) and 9.72 (s, 1H); HRMS calcd for (M+1) C₂₃H₂₄NO 330.450, found: 330.1836. Anal. Calcd. for C₂₃H₂₃ON: C, 83.86; H, 7.04; N, 4.25. Found: C, 83.64; H, 7.42; N, 4.19. HPLC on chiral stationary phase:(S,S) Pirkle-Whelk-O 1 column (250 x 4.6 mm I.D.), mobile phase: hexane/isopropanol (99.5:0.5, v/v), flow-rate: 1.5 ml/min, detection with UV detector at 210nm. Retention time of the desired S-isomer: 8.75 min., retention time of the R-enanatiomer 10.62 min.

### METHOD 2:

A solution of oxalyl chloride (8.4 ml, 0.096 mol) in dichloromethane (240 ml) was cooled to -74°C. A solution of DMSO (12.0 ml, 0.155 mol) in dichloromethane (50 ml) was then slowly added at a rate to maintain the temperature at -74°C (addition time ∼1.25 hr). The mixture was stirred for 5 min. followed by addition of a solution of βS-2-[bis(phenylmethyl)amino]benzene-propanol (0.074 mol) in 100 ml of dichloromethane (addition time -20 min., temp. -75°C to -68°C). The solution was stirred at -78°C for 35 minutes under a nitrogen atmosphere. Triethylamine (41.2 ml, 0.295 mol) was then added over 10 min. (temp. -78° to -68°C) upon which the ammonium salt procipitated. The cold mixture was stirred for 30 min. and then water (225 ml) was added. The dichloromethane layer was separated from the aqueous phase and washed with water, brine, dried over magnesium sulfate, filtered and concentrated. The residue was diluted with ethyl acetate and hexane and then filtered to further remove the ammonium salt. The filtrate was concentrated to give αS-[bis(phenylmethyl)amino]benzenepropanaldehyde. The aldehyde was carried on to the next step without purification.

### METHOD 3:

To a mixture of 1.0 g(3.0 moles) of βS-2-[bis(phenylmethyl)amino]benzenepropanol 0.531 g(4.53 mmoles) of N-methyl morpholine, 2.27 g of molecular sieves(4A) and 9.1 mL of acetonitrile was added 53 mg(0.15 mmoles) of tetrapropylammonium perruthenate(TPAP). The mixture was stirred for 40 minutes at room temperature and concentrated under reduced pressure. The residue was suspended in 15 mL of ethyl acetate, filtered through a pad of silica gel. The filtrate was concentrated under reduced pressure to give a product containing approximately 50% of αS-2-[bis(phenylmethyl)amino]benzene propanaldehyde as a pale yellow oil.

### METHOD 4:

To a solution of 1.0 g (3.02 mmoles) of βS-2-[bis(phenylmethyl)amino]benzenepropanol in 9.0 mL of toluene was added 4.69 mg(0.03 mmoles) of 2,2,6,6-tetramethyl-1-piperidinyloxy, free radical (TEMPO), 0.32g(3.11 mmoles) of sodium bromide, 9.0 mL of ethyl acetate and 1.5 mL of water. The mixture was cooled to 0 ^{º}C and an aqueous solution of 2.87 mL of 5% household bleach containing 0.735 g(8.75 mmoles) of sodium bicarbonate and 8.53 mL of water was added slowly over 25 minutes. The mixture was stirred at 0 ^{º}C for 60 minutes. Two more additions (1.44 mL each) of bleach was added followed by stirring for 10 minutes. The two phase mixture was allowed to separate. The aqueous layer was extracted twice with 20 mL of ethyl acetate. The combined organic layer was washed with 4.0 mL of a solution containing 25 mg of potassium iodide and water(4.0 mL), 20 mL of 10% aqueous sodium thiosulfate solution and then brine solution. The organic solution was dried over magnesium sulfate, filtered and concentrated under reduced pressure to give 1.34g of crude oil containing a small amount of the desired product aldehyde, αS-[bis(phenylmethyl)amino]benzenepropanaldehyde.

### METHOD 5:

Following the same procedures as described in Example 2 (Method 1) except 3.0 equivalents of sulfur trioxide pyridine complex was used and αS-[bis(phenylmethyl)amino]benzenepropanaldehyde was isolated in comparable yields.

### EXAMPLE 3

### N,N,αS-Tris(phenylmethyl)-2S-oxiranemethanamine

### METHOD 1:

A solution of αS-[Bis(phenylmethyl)amino]benzene-propanaldehyde (191.7 g, 0.58 mol) and chloroiodomethane (56.4 mL, 0.77 mol) in tetrahydrofuran (1.8 L) was cooled to -30 to -35°C (colder temperature such as -70°C also worked well but warmer temperatures are more readily achieved in large scale operations) in a stainless steel reactor under a nitrogen atmosphere. A solution of n-butyllithium in hexane (1.6 M, 365 mL, 0.58 mol) was then added at a rate that maintained the temperature below -25°C. After addition the mixture was stirred at -30 to -35°C for 10 minutes. More additions of reagents were carried out in the following manner: (1) additional chloroiodomethane (17 mL) was added, followed by n-butyllithium (110 mL) at < -25°C. After addition the mixture was stirred at -30 to -35°C for 10 minutes. This was repeated once. (2) Additional chloroiodomethane (8.5 mL, 0.11 mol) was added, followed by n-butyllithium (55 mL, 0.088 mol) at <-25°C. After addition the mixture was stirred at -30 to -35°C for 10 minutes. This was repeated 5 times. (3) Additional chloroiodomethane (8.5 mL, 0.11 mol) was added, followed by n-butyllithium (37 mL, 0.059 mol) at <-25°C. After addition the mixture was stirred at -30 to -35°C for 10 minutes. This was repeated once. The external cooling was stopped and the mixture warmed to ambient temp. over 4 to 16 hours when TLC (silica gel, 20% ethyl acetate/hexane) indicated that the reaction was completed. The reaction mixture was cooled to 10°C and quenched with 1452 g of 16% ammonium chloride solution (prepared by dissolving 232 g of ammonium chloride in 1220 mL of water), keeping the temperature below 23°C. The mixture was stirred for 10 minutes and the organic and aqueous layers were separated. The aqueous phase was extracted with ethyl acetate (2x 500 mL). The ethyl acetate layer was combined with the tetrahydrofuran layer. The combined solution was dried, over magnesium sulfate (220 g), filtered and concentrated on a rotary evaporator at 65°C. The brown oil residue was dried at 70°C in vacuo (0.8 bar) for 1 h to give 222.8 g of crude material. (The crude product weight was >100%. Due to the relative instability of the product on silica gel, the crude product is usually used directly in the next step without purification). The diastereomeric ratio of the crude mixture was determined by proton NMR: (2S)/(2R): 86:14. The minor and major epoxide diastereomers were characterized in this mixture by tic analysis (silica gel, 10% ethyl acetate/hexane), Rf = 0.29 & 0.32, respectively. An analytical sample of each of the diastereomers was obtained by purification on silica-gel chromatography (3% ethyl acetate/hexane) and characterized as follows:

### N,N,αS-Tris(phenylmethyl)-2S-oxiranemethanamine

¹H NMR (400 MHz, CDCl₃) ∂ 2.49 and 2.51 (AB-System, 1H, J_{AB} = 2.82), 2.76 and 2.77 (AB-System, 1H, J_{AB} = 4.03), 2.83 (m, 2H), 2.99 & 3.03 (AB-System, 1H, J_{AB} = 10.1 Hz), 3.15 (m, 1H), 3.73 & 3.84 (AB-System, 4H, J_{AB} = 14.00), 7.21 (m, 15H); ¹³C NMR (400 MHz,CDCl₃) ∂ 139.55, 129.45, 128.42, 128.14, 128.09, 126.84, 125.97, 60.32, 54.23, 52.13, 45.99, 33.76; HRMS calcd for C₂₄H₂₆NO (M+1) 344.477, found 344.2003.

### N,N,αS-Tris(phenylmethyl)-2R-oxiranemethanamine

¹H NMR (300 MHz, CDCl₃) ∂ 2.20 (m, 1H), 2.59 (m, 1H), 2.75 (m, 2H), 2.97 (m, 1H), 3.14 (m, 1H), 3.85 (AB-System, 4H), 7.25 (m, 15H).HPLC on chiral stationary phase: Pirkle-Whelk-O 1 column (250 x 4.6 mm I.D.), mobile phase: hexane/isopropanol (99.5:0.5, v/v), flow-rate: 1.5 ml/min, detection with UV detector at 210nm. Retention time of(8): 9.38 min., retention time of enanatiomer of (4): 13.75 min.

### METHOD 2:

A solution of the crude aldehyde 0.074 mol and chloroiodomethane (7.0 ml, 0.096 mol) in tetrahydrofuran (285 ml) was cooled to -78°C, under a nitrogen atmosphere. A 1.6 M solution of n-butyllithium in hexane (25 ml, 0.040 mol) was then added at a rate to maintain the temperature at -75°C (addition time - 15 min.). After the first addition, additional chloroiodomethane (1.6 ml, 0.022 mol) was added again, followed by n-butyllithium (23 ml, 0.037 mol), keeping the temperature at -75°C. The mixture was stirred for 15 min. Each of the reagents, chloroiodomethane (0.70 ml, 0.010 mol) and n-butyllithium (5 ml, 0.008 mol) were added 4 more times over 45 min. at -75°C. The cooling bath was then removed and the solution warmed to 22°C over 1.5 hr. The mixture was poured into 300 ml of saturated aq. ammonium chloride solution. The tetrahydrofuran layer was separated. The aqueous phase was extracted with ethyl acetate (1 x 300 ml). The combined organic layers were washed with brine, dried over magnesium sulfate, filtered and concentrated to give a brown oil (27.4 g). The product could be used in the next step without purification. The desired diastereomer can be purified by recrystallization at a subsequent step. The product could also be purified by chromatography.

### METHOD 3:

A solution of αS-[Bis(phenylmethyl)amino]benzene-propanaldehyde (178.84 g, 0.54 mol) and bromochloromethane (46 mL, 0.71 mol) in tetrahydrofuran (1.8 L) was cooled to -30 to -35°C (colder temperature such as -70°C also worked well but warmer temperatures are more readily achieved in large scale operations) in a stainless steel reactor under a nitrogen atmosphere. A solution of n-butyllithium in hexane (1.6 M, 340 mL, 0.54 mol) was then added at a rate that maintained the temperature below -25°C. After addition the mixture was stirred at -30 to -35°C for 10 minutes. More additions of reagents were carried out in the following manner: (1) additional bromochloromethane (14 mL) was added, followed by n-butyllithium (102 mL) at < -25°C. After addition the mixture was stirred at -30 to -35°C for 10 minutes. This was repeated once. (2) Additional bromochloromethane (7 mL, 0.11 mol) was added, followed by n-butyllithium (51 mL, 0.082 mol) at <-25°C. After addition the mixture was stirred at -30 to -35°C for 10 minutes. This was repeated 5 times. (3) Additional bromochloromethane (7 mL, 0.11 mol) was added, followed by n-butyllithium (51 mL, 0.082 mol) at <-25°C. After addition the mixture was stirred at -30 to -35°C for 10 minutes. This was repeated once. The external cooling was stopped and the mixture warmed to ambient temp. over 4 to 16 hours when TLC (silica gel, 20% ethyl acetate/hexane) indicated that the reaction was completed. The reaction mixture was cooled to 10°C and quenched with 1452 g of 16% ammonium chloride solution (prepared by dissolving 232 g of ammonium chloride in 1220 mL of water), keeping the temperature below 23°C. The mixture was stirred for 10 minutes and the organic and aqueous layers were separated. The aqueous phase was extracted with ethyl acetate (2x 500 mL). The ethyl acetate layer was combined with the tetrahydrofuran layer. The combined solution was dried over magnesium sulfate (220 g), filtered and concentrated on a rotary evaporator at 65°C. The brown oil residue was dried at 70°C in vacuo (0.8 bar) for 1 h to give 222.8 g of crude material;

### METHOD 4:

Following the same procedures as described in Example 3 (Method 3) except the reaction temperatures were at -20 ^{º}C. The resulting N,N,αS-tris(phenylmethyl)-2S-oxiranemethanamine was a diastereomeric mixture of lesser purity then that of Method 3.

### METHOD 5:

Following the same procedures as described in Example 3 (Method 3) except the reaction temperatures were at -70--78 ^{º}C. The resulting N,N,αS-tris(phenylmethyl)-2S-oxiranemethanamine was a diastereomeric mixture, which was used directly in the subsequent steps without purification.

### METHOD 6:

Following the same procedures as described in Example 3 (Method 3) except a continuous addition of bromochloromethane and n-butyllithium was used at -30 to -35 ^{º}C. After the reaction and work up procedures as decribed in Example 3 (Method 3), the desired N,N,αS-tris(phenylmethyl)-2S-oxiranemethanamine was isolated in comparable yields and purities.

### METHOD 7:

Following the same procedures as described in Example 3 (Method 2) except dibromomethane was used instead of chloroiodomethane. After the reaction and work up procedures as decribed in Example 3 (Method 2), the desired N,N,αS-tris(phenylmethyl)-2S-oxirane-methanamine was isolated.

### EXAMPLE 4

### 3S-[N,N-Bis(phenylmethyl)amino]-1-(2-methylpropyl)amino-4-phenylbutan-2R-ol

To a solution of crude N,N,αS-tris(phenylmethyl)-2S-oxiranemethanamine (388.5 g, 1.13mol) from Example 3 in isopropanol (2.7 L) (or ethyl acetate) was added isobutylamine (1.7 kgm, 23.1 mol) over 2 min. The temperature increased from 25 ^{º}C to 30 ^{º}C. The solution was heated to 82 ^{º}C and stirred at this temperature for 1.5 h. The warm solution was concentrated under reduced pressure at 65 ^{º}C. The brown oil residue was transferred to a 3-L flask and dried in vacuo (0.8 mm Hg) for 16 h to give 450 g of 3S-[N,N-bis(phenylmethyl)amino]-1-(2-methylpropyl)amino-4-phenylbutan-2R-ol as a crude oil. The product was used directly in the next step without purification. An analytical sample of the desired major diastereomeric product was obtained by purifying a small sample of crude product by silica gel chromatography (40% ethyl acetate/hexane). Tlc analysis: silica gel, 40% ethyl acetate/hexane; Rf = 0.28; HPLC analysis: ultrasphere ODS column, 25% triethylamine-/phosphate buffer pH 3/acetonitrile, flow rate 1 mL/min, UV detector; retention time 7.49 min.; HRMS calcd for C28H37N2O (M + 1) 417.616, found 417.2887.

An analytical sample of the minor diastereomeric product, 3S-(N,N-bis(phenylmethyl)amino]-1-(2-methylpropyl)amino-4-phenylbutan-2S-ol was also obtained by purifying a small sample of crude product by silica gel chromatography (40% ethyl acetate/hexane).

### EXAMPLE 5

### 3S-[N,N-Bis(phenylmethyl)amino]-1-(3-methylbutyl)amino-4-phenylbutan-2R-ol

Example 4 was followed using isoamylamine instead of isobutylamine to prepare 3S-[N,N-Bis(phenylmethyl)amino]-1-(3-methylbutyl)amino-4-phenylbutan-2R-ol and 3S-[N,N-Bis(phenylmethyl)amino]-1-(3-methylbutyl)amino-4-phenylbutan-2S-ol in comparable yields to that of Example 4. The crude amine was used in the next step without further purification.

### EXAMPLE 6

### N-[3S-[N,N-Bis(phenylmethyl)amino]-2R-hydroxy-4-phenylbutyl]-N'-(1,1-dimethylethyl)-N-(2-methylpropyl)urea

A solution of the crude 3S-[N,N-Bis(phenylmethyl)amino]-1-(2-methylpropyl)amino-4-phenylbutan-2R-ol (446.0 g, 1.1 mol) from Example 4 in tetrahydrofuran (6 L) (or ethyl acetate) was cooled to 8 ^{º}C. t-Butyl isocyanate (109.5 g, 1.1 mol) was then added to the solution of the amine from an addition funnel at a rate that maintained the temperature between 10-12 ^{º}C (addition time was about 10 min). The external cooling was stopped and the reaction was warmed to 18 ^{º}C after 30 min. The solution was transferred directly from the reactor to a rotary evaporator flask (10 L) through a teflon tube using vacuum and then concentrated. The flask was heated in a 50 ^{º}C water bath during the 2 h required for the distillation of the solvent. The brown residue was dissolved in ethyl acetate (3 L), washed with 5% aq citric acid solution (1 x 1.2 L), water (2 x 500 mL), brine (1 x 400 mL), dried over magnesium sulfate ( 200 g) and filtered. The volume of product solution was reduced to 671 mL over 2 h on a rotary evaporator at 50 ^{º}C. The concentrate was stirred and diluted with 1.6 L of hexane. The mixture was cooled to 12 ^{º}C and stirred for 15 hours. The product crystals were isolated by filtration, washed with 10% ethyl acetate/hexane (1 x 500 mL), hexane (1 x 200 mL) and dried in vacuo (2 mm) at 50 ^{º}C for 1 hour to give 248 g of N-[3S-[N,N-bis-(phenylmethyl)amino]-2R-hydroxy-4-phenylbutyl]-N'-(1,1- dimethylethyl)-N-(2-methylpropyl)-urea. The mother liquor and washes were combined and concentrated on a rotary evaporator to give 270 g of a brown oil. This material was dissolved in ethyl acetate (140 mL) at 50 ^{º}C and diluted with hexane (280 mL) and seeded with crystals of the first crop product (20 mg). The mixture was cooled in an ice bath and stirred for 1 h. The solid was isolated by filtration, washed with 10% ethyl acetate/hexane (1 x 200 mL) and dried in vacuo (2 mm) at 50 ^{º}C for 1 h to give 55.7 g of 11 as the second crop (49% overall yield). Mp 126 ^{º}C; [α]D25 = -59.0 ^{º} (c = 1.0, CH2Cl2), TLC: Rf 0.31 (silica gel, 25% ethyl acetate/hexane).

An analytical sample of the minor diasatereomer, N-[3S-[N,N-bis(phenylmethyl)amino]-2S-hydroxy-4-phenylbutyl]-N'-(1,1-dimethylethyl)-N-(2-methylpropyl)urea was isolated by silica-gel chromatography (10-15% ethyl acetate/hexane) in an earlier experiment and characterized.

### EXAMPLE 7

### N-[3S-[N,N-Bis(phenylmethyl)amino]-2R-hydroxy-4-phenylbutyl]-N'-(1,1-dimethylethyl)-N-(3-methylbutyl)urea

The crude product from Example 5 was reacted with t-butylisocyanate following the method of Example 6 to prepare N-[3S-[N,N-Bis(phenylmethyl)amino]-2R-hydroxy-4-phenylbutyl]-N'-(1,1-dimethylethyl)-N-(3-methylbutyl)urea and N-[3S-[N,N-Bis(phenylmethyl)amino]-2S-hydroxy-4-phenylbutyl]-N'-(1,1-dimethylethyl)-N-(3-methylbutyl)urea in comparable yields to that of Example 6.

### EXAMPLE 8

### N-[3S-Amino-2R-hydroxy-4-phenylbutyl]-N'-(1,1-dimethylethyl)-N-(2-methylpropyl)urea

N-[3S-[N,N-Bis(phenylmethyl)amino]-2R-hydroxy-4-phenylbutyl]-N'-(1,1-dimethylethyl)-N-(2-methylpropyl)urea (125.77 g, 0.244 mol) from Example 6 was dissolved in ethanol (1.5 L) (or methanol) and 20% palladium hydroxide on carbon (18.87 g) (or 4% palladium on carbon) was added to the solution under nitrogen. The mixture was stirred at ambient temperature under a hydrogen atmosphere at 60 psi for approximately 8 h. The catalyst was removed by filtration and the filtrate was concentrated to give 85 g of N-[3S-Amino-2R-hydroxy-4-phenylbutyl]-N'-(1,1-dimethylethyl)-N-(2-methylpropyl)urea as a colorless oil.

### EXAMPLE 9

### N-[3S-Amino-2R-hydroxy-4-phenylbutyl]-N'-(1,1-dimethylethyl)-N-(3-methylbutyl)urea

N-[3S-[N,N-Bis(phenylmethyl)amino]-2R-hydroxy-4-phenylbutyl]-N'-(1,1-dimethylethyl)-N-(3-methylbutyl)urea from Example 7 was hydrogenated following the method of Example 8 to prepare N-[3S-Amino-2R-hydroxy-4-phenylbutyl]-N'-(1,1-dimethylethyl)-N-(3-methylbutyl)urea in comparable yields to Example 8.

### EXAMPLE 10

### N-benzyl-L-phenylalaninol

### METHOD 1:

L-Phenylalaninol (89.51 g, 0.592 moles) was dissolved in 375 mL of methanol under inert atmosphere, 35.52 g (0.592 moles) of glacial acetic acid and 50 mL of methanol was added followed by a solution of 62.83 g (0.592 moles) of benzaldehyde in 100 mL of methanol. The mixture was cooled to approximately 15 ^{º}C and a solution of 134.6 g(2.14 moles) of sodium cyanoborohydride in 700 mL of methanol was added in approximately 40 minutes, keeping the temperature between 15 ^{º}C and 25 ^{º}C. The mixture was stirred at room temperature for 18 hours. The mixture was concentrated under reduced presssure and partitioned between 1 L of 2M ammonium hydroxide solution and 2 L of ether. The ether layer was washed with 1 L of 1M ammonium hydroxide solution, twice with 500 mL water, 500 mL of brine and dried over magnesium sulfate for 1 hour. The ether layer was filtered, concentrated under reduced pressure and the crude solid product was recrystallized from 110 mL of ethyl acetate and 1.3 L of hexane to give 115 g (81% yield) of N-benzyl-L-phenylalaninol as a white solid.

### METHOD 2:

L-Phenylalaninol (5 g, 33 mmoles) and 3.59 g (33.83 mmoles) of benzaldehyde were dissolved in 55 mL of 3A ethanol under inert atmosphere in a Parr shaker and the mixture was warmed to 60 ^{º}C for 2.7 hours. The mixture was cooled to approximately 25 ^{º}C and 0.99 g of 5% platinum on carbon was added and the mixture was hydrogenated at 60 psi of hydrogen and 40 ^{º}C for 10 hours. The catalyst was filtered off, the product was concentrated under reduced pressure and the crude solid product was recrystallized from 150 mL of heptane to give 3.83 g( 48 % yield) of N-benzyl-L-phenylalaninol as a white solid.

### EXAMPLE 11

### N-(t-Butoxycarbonyl)-N-benzyl-L-phenylalaninol

N-benzyl-L-phenylalaninol (2.9 g, 12 mmoles) from Example 10 was dissolved in 3 mL of triethylamine and 27 mL of methanol and 5.25 g(24.1 mmoles) of di-tert-butyl dicarbonate was added. The mixture was warmed to 60 ^{º}C for 35 minutes and concentrated under reduced pressure. The residue was dissolved in 150 mL of ethyl acetate and washed twice with 10 mL of cold(0-5 ^{º}C), dilute hydrochoric acid (pH 2.5 to 3), 15 mL of water, 10 mL of brine, dried over magnesium sulfate, filtered and concentrated under reduced pressure. The crude product oil was purified by silica gel chromatography (ethyl acetate: hexane, 12:3 as eluting solvent) to give 3.98 g (97% yield) of colorless oil.

### EXAMPLE 12

### N-(t-Butoxycarbonyl)-N-benzyl-L-phenylalaninal

### METHOD 1:

To a solution of 0.32 g(0.94 mmoles) of N-(t-Butoxycarbonyl)-N-benzyl-L-phenylalaninol from Example 11 in 2.8 mL of toluene was added 2.4 mg (0.015 mmoles) of 2,2,6,6-tetramethyl-1-piperidinyloxy, free radical (TEMPO), 0.1g (0.97 mmoles) of sodium bromide, 2.8 mL of ethyl acetate and 0.34 mL of water. The mixture was cooled to 0 ^{º}C and an aqueous solution of 4.2 mL of 5% household bleach containing 0.23 g (3.0 mL, 2.738 mmoles) of sodium bicarbonate was added slowly over 30 minutes. The mixture was stirred at 0 ^{º}C for 10 minutes. Three more additions (0.4 mL each) of bleach was added followed by stirring for 10 minutes after each addition to consume all the stating material. The two phase mixture was allowed to separate. The aqueous layer was extracted twice with 8 mL of toluene. The combined organic layer was washed with 1.25 mL of a solution containing 0.075 g of potassium iodide, sodium bisulfate(0.125 g) and water(1.1 mL), 1.25 mL of 10% aqueous sodium thiosulfate solution, 1.25 mL of pH 7 phosphate buffer and 1.5 mL of brine solution. The organic solution was dried over magnesium sulfate, filtered and concentrated under reduced pressure to give 0.32 g (100% yield) of N-(t-Butoxycarbonyl)-N-benzyl-L-phenylalaninal.

### METHOD 2:

To a solution of 2.38 g(6.98 mmoles) of N-(t-butoxycarbonyl)-N-benzyl-L-phenylalaninol from Example 11 in 3.8 mL (27.2 mmoles) of triethylamine at 10 ^{º}C was added a solution of 4.33 g (27.2 mmoles) of sulfur trioxide pyridine complex in 17 mL of dimethyl sulfoxide. The mixture was warmed to room temperature and stirred for one hour. Water (16 mL) was added and the mixture was extracted with 20 mL of ethyl acetate. The oragnic layer was washed with 20 mL of 5% citric acid, 20 mL of water, 20 mL of brine, dried over magnesium sulfate and filtered. The filtrate was, concentrated under reduced pressure to give 2.37 g(100% yield) of N-(t-Butoxycarbonyl)-N-benzyl-L-phenylalaninal.

### EXAMPLE 13

### N,αS-Bis(phenylmethyl)-N-(t-butoxycarbonyl)-2S-oxiranemethanamine

### METHOD 1:

A solution of 2.5 g (7.37 mmoles) of N-(t-butoxycarbonyl)-N-benzyl-L-phenylalaninal from Example 12 and 0.72 mL of chloroiodomethane in 35 mL of THF was cooled to -78 ^{º}C. A 4.64 mL of a solution of n-butyllithium (1.6 M in hexane, 7.42 mmoles) was added slowly, keeping the temperature below -70 ^{º}C. The mixture was stirred for 10 minutes between -70 to -75 ^{º}C. Two additional portions of 0.22 mL of chloroiodomethane and 1.4 mL of n-butyllithium was added sequentially and the mixture was stirred for 10 minutes between -70 to -75 ^{º}C after each addition. Four additional portions of 0.11 mL of chloroiodomethane and 0.7 mL of n-butyllithium was added sequentially and the mixture was stirred for 10 minutes between -70 to -75 ^{º}C after each addition. The mixture was warmed to room temperature for 3.5 hours. The product was quenched at below 5 ^{º}C with 24 mL of ice-cold water. The biphasic layers were separated and the aqueous layer was extracted twice with 30 mL of ethyl acetate. The combined organic layers was washed three times with 10 mL water, then with 10 mL brine, dried over sodium sulfate, filtered and concentrated under reduced pressure to give 2.8 g of a yellow crude oil. This crude oil (>100% yield) is a mixture of the diastereomeric epoxides N,αS-bis(phenylmethyl)-N-(t-butoxycarbonyl)-2S-oxiranemethanamine and N,αS-bis(phenylmethyl)-N-(t-butoxycarbonyl)-2R-oxiranemethanamine. The crude mixture is used directly in the next step without purification.

### METHOD 2:

To a suspension of 2.92 g (13.28 mmoles) of trimethylsulfoxonium iodide in 45 mL of acetonitrile was added 1.49 g (13.28 mmoles) of potassium t-butoxide. A solution of 3.0 g (8.85 mmoles) of N-(t-butoxycarbonyl)-N-benzyl-L-phenylalaninal from Example 12 in 18 mL of acetonitrile was added and the mixture was stirred at room temperature for one hour. The mixture was diluted with 150 mL of water and extracted twice with 200 mL of ethyl acetate. The organic layers were combined and washed with 100 mL water, 50 mL brine, dried over sodium sulfate, filtered and concentrated under reduced pressure to give 3.0 g of a yellow crude oil. The crude product was purified by silica gel chromatography (ethyl acetate/hexane: 1: 8 as eluting sovent) to give 1.02 g (32.7% yield) of a mixture of the two diastereomers N,αS-bis(phenylmethyl)-N-(t-butoxycarbonyl)-2S-oxiranemethanamine and N,αS-bis(phenylmethyl)-N-(t-butoxycarbonyl)-2R-oxiranemethanamine.

### METHOD 3:

To a suspension of 0.90 g (4.42 mmoles) of trimethylsulfonium iodide in 18 mL of acetonitrile was added 0.495 g (4.42 mmoles) of potassium t-butoxide. A solution of 1.0 g (2.95 mmoles) of N-(t-butoxycarbonyl)-N-benzyl-L-phenylalaninal from Example 12 in 7 mL of acetonitrile was added and the mixture was stirred at room temperature for one hour. The mixture was diluted with 80 mL of water and extracted twice with 80 mL of ethyl acetate. The organic layers were combined and washed with 100 mL water, 30 mL brine, dried over sodium sulfate, filtered and concentrated under reduced pressure to give 1.04 g of a yellow crude oil. The crude product was a mixture of the two diastereomers N,αS-bis(phenylmethyl)-N-(t-butoxycarbonyl)-2S-oxiranemethanamine and N,αS-bis(phenylmethyl)-N-(t-butoxycarbonyl)-2R-oxiranemethanamine.

### EXAMPLE 14

### 3S-[N-(t-Butoxycarbonyl)-N-(phenylmethyl)amino]-1-(2-methylpropyl)amino-4-phenylbutan-2R-ol

To a solution of 500 mg (1.42 mmoles) of the crude epoxide from Example 13 in 0.98 mL of isopropanol was added 0.71 mL (7.14 mmoles) of isobutylamine. The mixture was warmed to reflux at 85 ^{º}C to 90 ^{º}C for 1.5 hours. The mixture was concentrated under reduced pressure and the product oil was purified by silica gel chromatography (chloroform:methanol, 100:6 as eluting solvents) to give 330 mg of 3S-[N-(t-butoxycarbonyl)-N-(phenylmethyl)amino]-1-(2-methylpropyl)amino-4-phenylbutan-2R-ol as a colorless oil (54.5% yield). 3S-[N-(t-Butoxycarbonyl)-N-(phenylmethyl)amino]-1-(2-methylpropyl)amino-4-phenylbutan-2S-ol was also isolated. When purified N,αS-bis(phenylmethyl)-N-(t-butoxycarbonyl)-2S-oxiranemethanamine was used as starting material, 3S-[N-(t-butoxycarbonyl)-N-(phenylmethyl)amino]-1-(2-methylpropyl)amino-4-phenylbutan-2R-ol was isolated after purification by chromatography in an 86% yield.

### EXAMPLE 15

### N-[3S-[N-(t-Butoxycarbonyl)-N-(phenylmethyl)amino]-2R-hydroxy-4-phenylbutyl]-N'-(1,1-dimethylethyl)-N-(2-methylpropyl)urea

To a solution of 309 mg (0.7265 mmoles) of 3S-[N-(t-butoxycarbonyl)-N-(phenylmethyl)amino]-1-(2- methylpropyl)amino-4-phenylbutan-2R-ol from Example 14 in 5 mL of THF was added 0.174 mL(1.5 mmoles) of t-butylisocyanate. The mixture was stirred at room temperature for 1.5 hours. The product was concentrated under reduced pressure to give 350 mg (92% yield) of a white solid crude product. The crude product was purified by silica gel chromatography (ethyl acetate/hexane: 1: 4 as eluting solvents) to give 324 mg of N-[3S-[N-(t-butoxycarbonyl)-N-(phenylmethyl)amino]-2R-hydroxy-4-phenylbutyl]-N'-(1,1-dimethylethyl)-N-(2-methylpropyl)urea as a white solid (85.3% yield).

### EXAMPLE 16

### 3S-(N-t-Butoxycarbonyl)amino-4-phenylbutan-1,2R-diol

To a solution of 1 g (3.39 mmoles) of 2S-(N-t-butoxycarbonyl)amino-1S-hydroxy-3-phenylbutanoic acid (commercially available from Nippon Kayaku, Japan) in 50 mL of THF at 0 ^{º}C was added 50 mL of borane-THF complex (liquid,1.0 M in THF), keeping the temperatures below 5 °C. The reaction mixture was warmed to room temperature and stirred for 16 hours. The mixture was cooled to 0 °C and 20 mL of water was added slowly to destroy the excess BH₃ and to quench the product mixture, keeping the temperature below 12 °C. The quenched mixture was stirred for 20 minutes and concentrated under reduced pressure. The product mixture was extracted three times with 60 mL of ethyl acetate. The organic layers were combined and washed with 20 mL of water, 25 mL of saturated sodium chloride solution and concentrated under reduced pressure to give 1.1 g of crude oil. The crude product was purified by silica gel chromatography (chloroform/methanol, 10:6 as eluting solvents) to give 900 mg (94.4% yield) of 3S-(N-t-butoxycarbonyl)amino-4-phenylbutan-1,2R-diol as a white solid.

### EXAMPLE 17

### 3S-(N-t-Butoxycarbonyl)amino-2R-hydroxy-4-phenylbut-1-yl Toluenesulfonate

To a solution of 744.8 mg (2.65 mmoles) of 3S-(N-t-butoxycarbonyl)amino-4-phenylbutan-1,2R-diol from Example 16 in 13 mL of pyridine at 0 ^{º}C was added 914 mg of toluenesulfonyl chloride in one portion. The mixture was stirred at 0 °C to 5 °C for 5 hours. A mixture of 6.5 mL of ethyl acetate and 15 mL of 5% aqueous sodium bicarbonate solution was added to the reaction mixture and stirred for 5 minutes. The product mixture was extracted three times with 50 mL of ethyl acetate. The organic layers were combined and washed with 15 mL of water, 10 mL of saturated sodium chloride solution and concentrated under reduced pressure to give about 1.1 g of a yellow chunky solid. The crude product was purified by silica gel chromatography (ethyl acetate/hexane 1:3 as eluting solvents) to give 850 mg (74% yield) of 3S-(N-t-butoxycarbonyl)amino-2R-hydroxy-4-phenylbut-1-yl toluenesulfonate as a white solid.

### EXAMPLE 18

### 3S-[N-(t-Butoxycarbonyl)amino]-1-(2-methylpropyl)amino-4-phenylbutan-2R-ol

To a solution of 90 mg (0.207 mmoles) of 3S-(N-t-butoxycarbonyl)amino-2R-hydroxy-4-phenylbut-1-yl toluenesulfonate from Example 17 in 0.143 mL of isopropanol and 0.5 mL of toluene was added 0.103 mL (1.034 mmoles) of isobutylamine. The mixture was warmed to 80 to 85 °C and stirred for 1.5 hours. The product mixture was concentrated under reduced pressure at 40 to 50 °C and purified by silica gel chromatography (chloroform/methanol, 10:1 as eluting solvents) to give 54.9 mg (76.8% yield) of 3S-[N-(t-butoxycarbonyl)amino]-1-(2-methylpropyl)amino-4-phenylbutan-2R-ol as a white solid.

### EXAMPLE 19

### N-[3S-[N-(t-Butoxycarbonyl)amino]-2R-hydroxy-4-phenylbutyl]-N'-(1,1-dimethylethyl)-N-(2-methylpropyl)urea

To a solution of 0.1732 g (0.516 mmoles) of 3S-[N-(t-butoxycarbonyl)amino]-1-(2-methylpropyl)amino-4-phenylbutan-2R-ol from Example 18 in 5 mL of ethyl acetate at 0 ^{º}C was added 1.62 mL (12.77 mmoles) of t-butylisocyanate and the mixture was stirred for one hour. The product was concentrated under reduced pressure and purified by silica gel chromatography (chloroform/methanol, 100:1.5 as eluting solvents) to give 96 mg (42.9 % yield) of N-[3S-[N-(t-butoxycarbonyl)amino]-2R-hydroxy-4-phenylbutyl]-N'-(1,1-dimethylethyl)-N-(2-methylpropyl)urea as a white solid.

### EXAMPLE 20

### N-[3S-amino-2R-hydroxy-4-phenylbutyl]-N'-(1,1-dimethylethyl)-N-(2-methylpropyl)urea

To a solution of 10 mg (0.023 mmoles) of N-[3S-[N-(t-butoxycarbonyl)amino]-2R-hydroxy-4-phenylbutyl]-N'-(1,1-dimethylethyl)-N-(2-methylpropyl)urea from Example 19 in 1 mL of methanol at 0 ^{º}C was added 1.05 mL of a 4M hydrogen chloride in methanol and the mixture was stirred at room temperature for 45 minutes. The product was concentrated under reduced pressure. The residue was dissolved 5 mL of methanol and concentrated under reduced pressure. This operation was repeated three times to remove water form the product, after which 8.09 mg (95.2% yield) of N-[3S-amino-2R-hydroxy-4-phenylbutyl]-N'-(1,1-dimethylethyl)-N-(2- methylpropyl)urea hydrochloride salt was obtained as a white solid.

### EXAMPLE 21

### 3S-(N,N-Dibenzyl)amino-2S-hydroxy-4-phenylbutyronitrile, O-trimethylsilyl ether

To a solution of 24.33 g (73.86 mmol) of 2S-(N,N-dibenzyl)amino-3-phenylpropanal in 740 mL of anhydrous methylene chloride at -20 C under a nitrogen atmosphere, was added 11.8 mL (8.8 g, 88.6 mmol) of trimethylsilylcyanide, then 19.96 g (88.6 mmol) of anhydrous zinc bromide. After 4 hours at -15 C, and 18 hours at room temperature, the solvent was removed under reduced pressure, ethyl acetate was added, washed with water, brine, dried over magnesium sulfate, filtered and concentrated to afford 31.3 g of a brown oil, which was identified as a 95:5 mixture of 3S-(N,N-dibenzyl)amino-2S-hydroxy-4-phenylbutyronitrile, O-trimethylsilyl ether, m/e=429(M+H) and 3S-(N,N-dibenzyl)amino-2R-hydroxy-4-phenylbutyronitrile, O-trimethylsilyl ether, respectively.

### EXAMPLE 22

### 3S-(N,N-Dibenzyl)amino-2S-hydroxy-4-phenylbutyronitrile

A solution of 10.4 g (24.3 mmol) of the crude 95:5 mixture of 3S-(N,N-dibenzyl)amino-2S-hydroxy-4-phenylbutyronitrile, O-trimethylsilyl ether, and 3S-(N,N-dibenzyl)amino-2R-hydroxy-4-phenylbutyronitrile, O-trimethylsilyl ether from Example 21 in 40 mL of methanol, was added to 220 mL of 1N hydrochloric acid with vigorous stirring. The resulting solid was collected, dissolved in ethyl acetate, washed with aqueous sodium bicarbonate, brine, dried over anhydrous magnesium sulfate, filtered and concentrated to afford 8.04 g of crude product. This was recrystallized from ethyl acetate and hexane to afford pure 3S-(N,N-dibenzyl)amino-2S-hydroxy-4-phenylbutyronitrile, m/e=357 (M+H).

### EXAMPLE 23

### 3S-(N,N-Dibenzyl)amino-2R-hydroxy-4-phenylbutylamine

### METHOD 1:

A solution of 20.3 g (47.3 mmol) of the crude 95:5 mixture of 3S-(N,N-dibenzyl)amino-2S-hydroxy-4-phenylbutyronitrile, O-trimethylsilyl ether, and 3S-(N,N-dibenzyl)amino-2R-hydroxy-4-phenylbutyronitrile, O-trimethylsilyl ether from Example 22 in 20 mL of anhydrous diethyl ether, was added to 71 mL (71 mmol) of a 1M solution of lithium aluminum hydride in diethyl ether at reflux. After the addition, the reaction was refluxed for 1 hour, cooled to 0C, and quenched by the careful addition of 2.7 mL of water, 2.7 mL of 15% aqueous sodium hydroxide, and 8.1 mL of water. The resulting solids were removed by filtration and the filtrate washed with water, brine, dried over magnesium sulfate, filtered and concentrated to afford 13.8 g of crude material, which was recrystallized from tetrahydrofuran and isooctane to afford 10.6 g of 3S-(N,N-dibenzyl)amino-2R-hydroxy-4-phenylbutylamine, mp 46-49 C, m/e=361 (M+H), which was contaminated by approximately 2% of 3S-(N,N-dibenzyl)amino-2S-hydroxy-4-phenylbutylamine.

### METHOD 2:

To 15.6 mL (60.4 mmol) of 70% sodium bis(methoxyethoxy)aluminum hydride in toluene, was added 15mL of anhydrous toluene, and then after cooling to 0C, a solution of 20.0g (46 mmol) of the crude 95:5 mixture of 3S-(N,N-dibenzyl)amino-2S-hydroxy-4-phenylbutyronitrile, O-trimethylsilyl ether, and 3S-(N,N-dibenzyl)amino-2R-hydroxy-4-phenylbutyronitrile, O-trimethylsilyl ether from Example 22 in 10 mL of anhydrous toluene, at a rate so as to maintain the temperature below 15°C. After 2.5 hours at room temperature, the reaction was quenched by the careful addition of 200 mL of 5% aqueous sodium hydroxide. The solution was diluted with ethyl acetate, washed with 5% sodium hydroxide, sodium tartrate solution, brine, dried over magnesium sulfate, filtered and concentrated to afford 16.6 g of crude product, which was assayed by HPLC and shown to contain 87% of 3S-(N,N-dibenzyl)amino-2R-hydroxy-4-phenylbutylamine.

### EXAMPLE 24

### N-[3S-(N,N-Dibenzyl)amino-2R-hydroxy-4-phenylbutyl]-N'-(1,1-dimethylethyl)-N-(3-methylbutyl)urea

Step 1: To a solution of 1.0 g (2.77 mmol) of 3S-(N,N-dibenzyl)amino-2R-hydroxy-4-phenylbutylamine from Example 23 in 4.6 mL of ethanol, was added 0.3 mL (0.24g, 2.77 mmol) of isovaleraldehyde. After 1 hour at room temperature, the ethanol was removed under reduced pressure, 4 mL of ethyl acetate was added and the solution purged with nitrogen. To the solution was added 360 mg of 5% platinum on carbon catalyst, the solution purged with 40 psig of hydrogen and then maintained under 40 psig of hydrogen for 20 hours. The solution was purged with nitrogen, the catalyst removed by filtration and the solvent removed under reduced pressure to afford 473 mg of the crude product.
Step 2: The crude product from Step A was directly dissolved in 5.4 mL of ethyl acetate and 109 mg (1.1 mmol) of tertiary-butyl isocyanate was added. After 1 hour at room temperature, the solution was washed with 5% citric acid, brine, dried over magnesium sulfate, filtered and concentrated to afford 470 mg of crude product. The crude product was recrystallized from ethyl acetate and isooctane to afford 160 mg of N-[3S-(N,N-Dibenzyl)amino-2R-hydroxy-4-phenylbutyl]-N'-(1,1-dimethylethyl)-N-(3-methylbutyl)urea, mp 120.4-121.7°C, m/e=530 (M+H).

From the foregoing detailed description, one skilled in the art can easily ascertain the essential characteristics of this invention.

## Claims

1. A method of preparing a substituted aminoalkohol of the formula 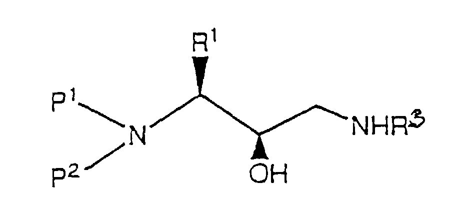
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom to which they are bonded to form a heterocyclic ring system containing said nitrogen atom as a ring member;
wherein R¹ is selected from alkyl, aryl, cycloalkyl, cycloalkylalkyl and aralkyl, which are optionally substituted with a group selected from alkyl, halo, NO₂, OR⁹ and SR⁹, wherein R⁹ is selected from hydrogen and alkyl;
and wherein R³ represents hydrogen, alkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, heteroaralkyl, aminoalkyl and mono- and disubstituted aminoalkyl. radicals, wherein said substituents are selected from alkyl, aryl, aralkyl, cycloalkyl, cycloalkylalkyl, heteroaryl, heteroaralkyl, heterocycloalkyl, and heterocycloalkylalkyl radicals, or in the case of a disubstituted aminoalkyl radical, said substituents along with the nitrogen atom to which they are attached, form a heterocycloalkyl or a heteroaryl radical;
and wherein any of the foregoing groups of P¹, P² and R³ may be substituted at one or more substitutable positions with one or more radicals independently selected from halo, alkyl of C₁-C₈, alkoxy, hydroxy, nitro, alkenyl, amino, alkylamino, acylamino and acyl; or a pharmaceutically-acceptable salt thereof; said method comprising the step of forming a N,N-protected aminoalcohol of the formula (II) 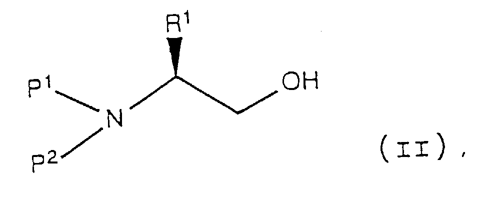 oxidizing said N,N-protected aminoalcohol of formula (II) to a chiral N,N-protected aminoaldehyde of the formula (III) 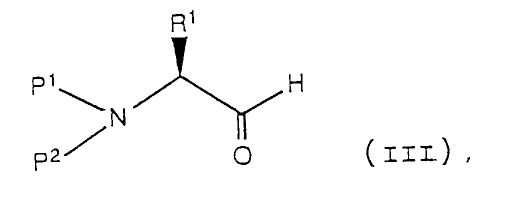 and diastereoselectively forming an aminoepoxide from said aminoaldehyde with an organometallic methylene-adding reagent selected from a halomethyllithium compound in an appropriate solvent and reacting said aminoepoxide with an amine of the formula R³NH₂ wherein R³ is as defined above to form said substituted aminoalcohol.

2. A method according to Claim 1 wherein an aminoepoxide compound of Formula I is prepared: 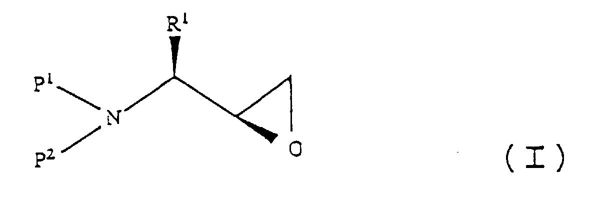
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom or Formula I to form a heterocyclic ring system containing said nitrogen atom as a ring member;
and wherein R¹ is selected from alkyl, aryl, cycloalkyl, cycloalkylalkyl and aralkyl, which are optionally substituted with a group selected from alkyl, halo, NO₂, OR⁹ and SR⁹, wherein R⁹ is selected from hydrogen and alkyl;
and wherein any of the foregoing groups of P¹ and P² may be substituted at one or more substitutable-positions with one or more radicals independently selected from halo, alkyl of C₁-C₈, alkoxy, hydroxy, nitro, alkenyl, amino, alkylamino, acylamino and acyl; or a pharmaceutically-acceptable salt thereof;
said method comprising the steps of forming a N,N-protected aminoalcohol of the formula (II) 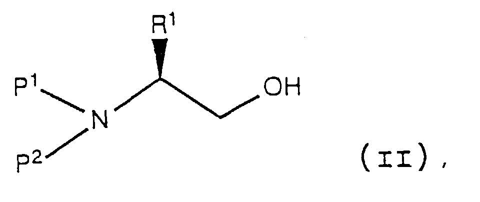 oxidizing said N,N-protected aminoalcohol of formula (II) to a chiral N,N-protected aminoaldehyde of the formula (III) 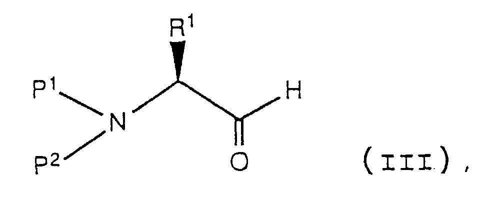 and diastereoselectively forming an aminoepoxide from said aminoaldehyde with an organometallic methylene-adding reagent selected from a halomethyllithium compound in an appropriate solvent.

3. The method of Claim 2 wherein P¹ and P² independently are selected from carbobenzoxy, t-butoxycarbonyl, acetyl, butyryl, benzoyl, isobutyloxycarbonyl, allyl, 1,2-bis(dimethylsilyl)ethane, 1,2-bis(dimethylsilyl)benzene, substituted benzoyl, trifluoroacetyl, trichloroacetyl, phthaloyl, benzyl, ortho-methylbenzyl, trityl, 1,2-bis(methylene)benzene, benzhydryl, phenethyl, phenpropyl, phenyl, naphthalenyl, indenyl, anthracenyl, durenyl, 9-(9-phenyl-fluoroenyl) and phenanthrenyl, wherein further P¹ and P² may be taken together to form with the nitrogen atom of Formula I a radical selected from phthalimide, succinimide and maleimide, and wherein any of the foregoing groups of P¹ and P² may be substituted at one or more substitutable positions with one or more radicals independently selected from halo, alkyl of C₁-C₈, alkoxy, hydroxy, nitro, alkenyl, amino, alkylamino, acylamino and acyl; or a pharmaceutically-acceptable salt thereof.

4. The method of Claim 2 wherein P¹ and P² are independently selected from aralkyl, 1-2-bis(methylene)benzene and aralkyl substituted with one or more radicals independently selected from halo, alkyl of C₁-C₈, alkoxy, hydroxy, nitro, alkenyl, amino, alkylamino, acylamino and acyl; and wherein R¹ is aralkyl; or a pharmaceutically-acceptable salt thereof.

5. A method according to Claim 1 wherein a protected chiral alpha-amino epoxide of Formula I: 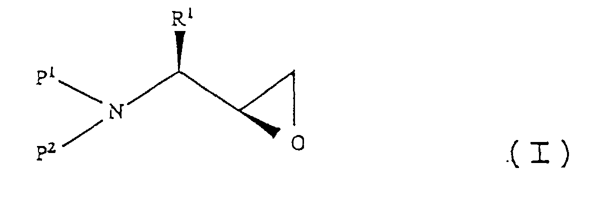
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom of Formula I to form a heterocyclic ring system containing said nitrogen atom as a ring member;
and wherein R¹ is selected from alkyl, aryl, cycloalkyl, cycloalkylalkyl and aralkyl, which are optionally substituted with a group selected from alkyl, halo, NO₂, OR⁹ and SR⁹, wherein R⁹ is selected from hydrogen and alkyl;
and wherein any of the foregoing groups of P¹ and P² may be substituted at one or more substitutable positions with one or more radicals independently selected from halo, alkyl of C₁-C₈, alkoxy, hydroxy, nitro, alkenyl, amino, alkylamino, acylamino and acyl; or a pharmaceutically-acceptable salt thereof;
is diastereoselectively and enantioselectively prepared by treating a protected aminoaldehyde substrate with an organometallic methylene-adding reagent in an appropriate solvent.

6. The method of Claim 5 wherein the organometallic methylene-adding reagent is a halomethyllithium generated in situ.

7. The method of Claim 6 wherein at least an equimolar amount of organometallic methylene-adding reagent is added to the aminoaldehyde.

8. The method of Claim 5 wherein the halomethyllithium is formed through the addition of an organolithium reagent with a dihalomethane.

9. The method of Claim 5 wherein the dihalomethane is selected from bromochloromethane, chloroiodomethane, dibromomethane, diiodomethane and bromofluoromethane.

10. The method of Claim 5 wherein the halomethyllithium is added to the amino aldehyde at a temperature in a range of about -80°C to about 0°C.

11. The method of Claim 5 wherein the halomethyllithium is added to the amino aldehyde at a temperature in a range of about -40°C and -15°C.

12. The diastereoselective method of preparing protected chiral alpha-amino epoxides of Claim 5 wherein the amino aldehyde is alpha-S-[bis(phenylmethyl)amino]benzenepropanaldehyde.

13. A method according to Claim 1 wherein a protected alpha-aminoaldehyde of Formula III: 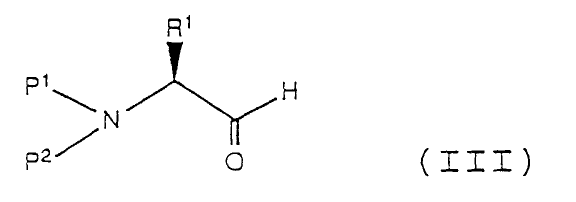
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom of Formula III to form a heterocyclic ring system containing said nitrogen atom as a ring member;
and wherein R¹ is selected from alkyl, aryl, cycloalkyl, cycloalkylalkyl and aralkyl, which are optionally substituted with a group selected from alkyl, halo, NO₂, OR⁹ and SR⁹, wherein R⁹ is selected from hydrogen and alkyl;
and wherein any of the foregoing groups of P¹ and P² may be substituted at one or more substitutable positions with one or more radicals independently selected from halo, alkyl of C₁-C₈, alkoxy, hydroxy, nitro, alkenyl, amino, alkylamino, acylamino and acyl; or a pharmaceutically-acceptable salt thereof;
is prepared by treating at a temperature of 0°C to 30°C a protected aminoalcohol with an oxidizing agent.

14. The method of Claim 13 wherein at least an equimolar amount of said oxidizing agent is added to the protected aminoalcohol.

15. The method of Claim 13 wherein said oxidizing agent is selected from sulfur trioxide:pyridine complex, acetyl chloride/dimethyl sulfoxide, acetyl anhydride/dimethyl sulfoxide, trifluoroacetyl chloride/dimethyl sulfoxide, toluenesulfonyl bromide/dimethyl sulfoxide, phosphorous pentachloride/dimethyl sulfoxide and isobutylchlorformate/dimethyl sulfoxide.

16. The method of Claim 13 wherein said oxidizing agent is sulfur trioxide:pyridine complex in an appropriate solvent.

17. The method of Claim 13 wherein the reaction temperature is between about 15°C and about 30°C.

18. The method of Claim 13 wherein the protected aminoalcohol is (Bis(phenylmethyl)amino]benzenepropanol.

19. A method according to Claim 1 wherein a protected chiral alpha-amino alcohol of the formula: 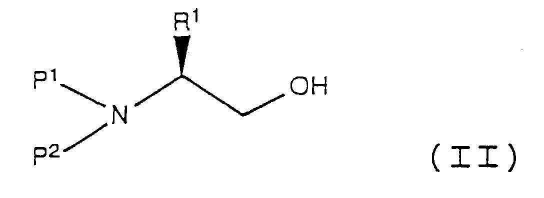
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom of Formula II to form a heterocyclic ring system containing said nitrogen atom as a ring member;
and wherein R¹ is selected from alkyl, aryl, cycloalkyl, cycloalkylalkyl and arylalkyl, which are optionally substituted at one or more substitutable positions with a group selected from alkyl, halo, NO₂, OR⁹ and SR⁹, wherein R⁹ is selected from hydrogen and alkyl;
and wherein any of the foregoing groups of P¹ and P² may be substituted with one or more radicals independently selected from halo, alkyl of C₁-C₈, alkoxy, hydroxy, nitro, alkenyl, amino, alkylamino, acylamino and acyl; or a pharmaceutically-acceptable salt thereof;
is prepared by treating said aminoalcohol with an alkylating agent.

20. The method of Claim 19 wherein the aminoalcohol is L-phenylalaninol.

21. A method according to Claim 1 wherein a protected chiral alpha-amino alcohol of Formula II: 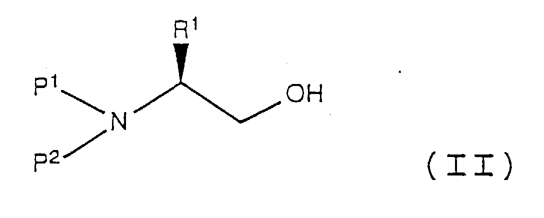
wherein P¹ and P² independently are selected from acyl, aralkyl, alkenyl, silyl, aralkoxycarbonyl, alkoxycarbonyl and cycloalkenylalkyl;
wherein further P¹ and P² may be taken together with the nitrogen atom of Formula II to form a heterocyclic ring system containing said nitrogen atom as a ring member;
and wherein R¹ is selected from alkyl, aryl, cycloalkyl, cycloalkylalkyl and arylalkyl, which are optionally substituted at one or more substitutable positions with a group selected from alkyl, halo, NO₂, OR⁹ and SR⁹, wherein R⁹ is selected from hydrogen and alkyl;
and wherein any of the foregoing groups of P¹ and P² may be substituted one or more radicals independently selected from halo, alkyl of C₁-C₈, alkoxy, hydroxy, nitro, alkenyl, amino, alkylamino, acylamino and acyl; or a pharmaceutically-acceptable salt thereof;
is prepared by treating an amino acid with an alkylating agent to form a protected amino acid, and forming a protected aminoalcohol by treating said protected amino acid with a reducing agent in a suitable solvent.

22. The method of Claim 21 wherein the reducing agent is diisobutylaluminum hydride.

23. The method of Claim 21 wherein said amino acid is L-phenylalanine.

## Patentansprüche

1. Ein Verfahren zur Herstellung eines substituierten Aminoalkohols der Formel 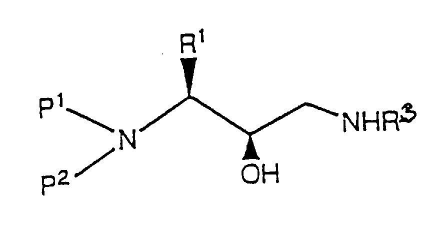
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin R³ Wasserstoff, Alkyl, Alkenyl, Alkynyl, Hydroxyalkyl, Alkoxyalkyl, Cycloalkyl, Cycloalkylalkyl, Heterocycloalkyl, Heteroaryl, Heterocycloalkylalkyl, Aryl, Aralkyl, Heteroaralkyl, Aminoalkyl und mono- und disubstituierte Aminoalkylreste bedeutet, worin diese Substituenten ausgewählt sind aus Alkyl-, Aryl-, Aralkyl-, Cycloalkyl-, Cycloalkylalkyl-, Heteroaryl-, Heteroaralkyl-, Heterocycloalkyl- und Heterocycloalkylalkylresten, oder im Falle eines disubstitierten Aminoalkylrestes diese Substituenten zusammen mit dem Stickstoffatom, an das sie gebunden sind, einen Heterocycloalkyl- oder Heteroarylrest bilden
und worin jede der vorstehenden Gruppen P¹, P² und R³ substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch verträgliches Salz davon; wobei dieses Verfahren folgende Stufen umfaßt: Bildung eines N,N-geschützten Aminoalkohols der Formel (II) 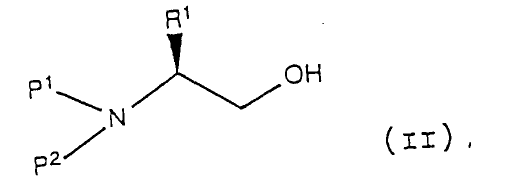 Oxidieren dieses N,N-geschützten Aminoalkohols der Formel (II) zu einem chiralen N,N-geschützten Aminoaldehyds der Formel (III) 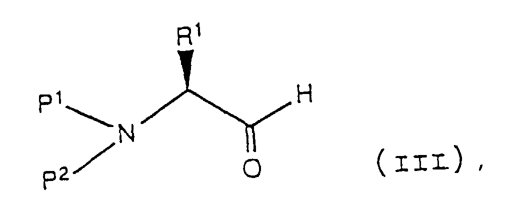 und diastereoselective Bildung eines Aminoepoxids aus diesem Aminoaldehyd mit einem organometallischen Methylenanlagerungsreagenz ausgewählt aus einer Halomethyllithiumverbindung in einem geeigneten Lösungsmittel und Umsetzung dieses Aminoepoxids mit einem Amin der Formel R³NH₂, worin R³ vorstehende Bedeutung hat, unter Bildung dieses substituierten Aminoalkohols.

2. Ein Verfahren gemäß Anspruch 1, worin eine Aminoepoxidverbindung hergestellt wird der Formel I: 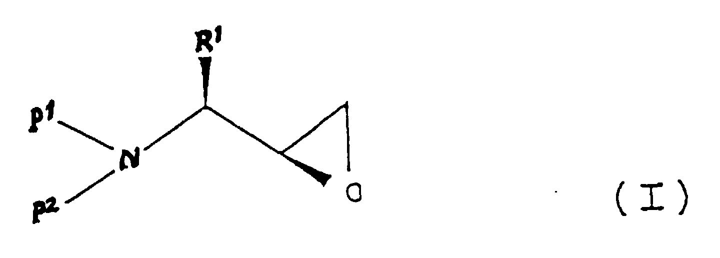
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin jede der vorstehenden Gruppen P¹ und P² substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch vertragliches Salz davon; wobei dieses Verfahren folgende Stufen umfaßt: Bildung eines N,N-geschützten Aminoalkohols der Formel (II) 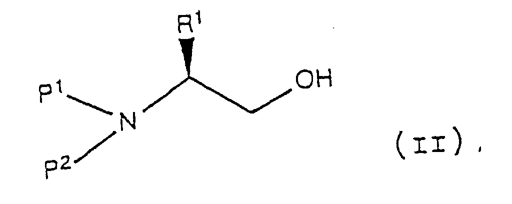 Oxidieren dieses N,N-geschützten Aminoalkohols der Formel (II) zu einem chiralen N,N-geschützten Aminoaldehyds der Formel (III) 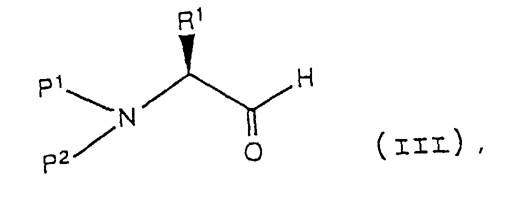 und diastereoselective Bildung eines Aminoepoxids aus diesem Aminoaldehyd mit einem organometallischen Methylenanlagerungsreagenz ausgewählt aus einer Halomethyllithiumverbindung in einem geeigneten Lösungsmittel.

3. Das Verfahren des Anspruchs 2, worin P¹ und P² unabhängig voneinander ausgewählt sind aus Carbobenzoxy, t-Butoxycarbonyl, Acetyl, Butyryl, Benzoyl, Isobutyloxycarbonyl, Allyl, 1,2-bis(Dimethylsilyl)ethan, 1,2-bis(Dimethylsilyl)benzol, substituiertes Benzoyl, Trifluoracetyl, Trichloracetyl, Phthaloyl, Benzyl, ortho-Methylbenzyl, Trityl, 1,2-bis(Methylen)benzol, Benzhydryl, Phenethyl, Phenpropyl, Phenyl, Naphthalenyl, Indenyl, Antracenyl, Durenyl, 9-(9-Phenyl-fluorenyl) und Phenantrenyl, worin außerdem P¹ und P² zusammengenommen werden können unter Bildung mit dem Stickstoffatom der Formel I eines Restes ausgewählt aus Phthalimid, Succinimid und Maleimid und worin jede der vorstehenden Gruppen von P¹ und P² substituiert sein kann an einer oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl oder ein pharmazeutisch vertragliches Salz davon.

4. Das Verfahren des Anspruchs 2, worin P¹ und P² unabhängig voneinander ausgewählt sind aus Aralkyl, 1-2-bis(Methylen)benzol und Aralkyl, substituiert mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl und worin R¹ Aralkyl ist oder ein pharmazeutisch verträgliches Salz davon.

5. Ein Verfahren gemäß Anspruch 1, worin ein geschütztes chirales Alpha-Aminoepoxid der Formel I: 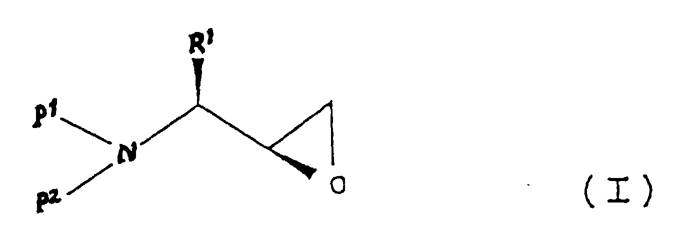
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin jede der vorstehenden Gruppen P¹ und P² substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch verträgliches Salz davon;
diastereoselectiv und enantioselectiv hergestellt wird durch Behandlung eines geschützten Aminoaldehydsubstrats mit einem organometallischen Methylenanlagerungsreagenz in einem geeigneten Lösungsmittel.

6. Das Verfahren des Anspruchs 5, worin das organometallische Methylenanlagerungsreagenz ein in situ erzeugtes Halomethyllithium ist.

7. Das Verfahren des Anspruchs 6, worin mindestens eine equimolare Menge des organometallischen Methylenanlagerungsreagenz dem Aminoaldehyd zugesetzt wird.

8. Das Verfahren des Anspruchs 5, worin das Halomethyllithium gebildet wird durch Zugabe eines Organolithiumreagenz mit einem Dihalomethan.

9. Das Verfahren des Anspruchs 5, worin das Dihalomethan ausgewählt ist aus Bromchlormethan, Chloriodmethan, Dibrommethan, Diiodmethan und Bromfluormethan.

10. Das Verfahren des Anspruchs 5, worin das Halomethyllithium zugesetzt wird zu dem Aminoaldehyd bei einer Temperatur im Bereich von etwa -80°C bis etwa 0°C.

11. Das Verfahren des Anspruchs 5, worin das Halomethyllithium zugesetzt wird zu dem Aminoaldehyd bei einer Temperatur im Bereich von etwa -40°C und -15°C.

12. Das diastereoselective Verfahren zur Herstellung der geschützten chiralen Alpha-Aminoepoxide des Anspruchs 5, worin das Aminoaldehyd Alpha-S[bis(phenylmethyl)amino]-benzolpropanaldehyd ist.

13. Ein Verfahren gemäß Anspruch 1 worin ein geschütztes Alpha-Aminoaldehyd der Formel III 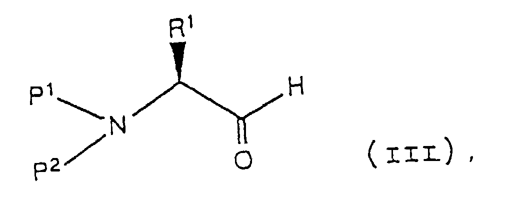
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin jede der vorstehenden Gruppen P¹ und P² substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch verträgliches Salz davon;
hergestellt wird durch Behandlung bei einer Temperatur von 0°C bis 30°C eines geschützten Aminoalkohols mit einem Oxidationsmittel.

14. Das Verfahren des Anspruchs 13, worin mindestens eine equimolare Menge dieses Oxidationsmittels dem geschützten Aminoalkohol zugesetzt wird.

15. Das Verfahren des Anspruchs 13, worin dieses Oxidationsmittel ausgewählt ist aus einem Schwefeltrioxid Pyridincomplex, Acetylchlorid/Dimethylsulfoxid, Acetylanhydrid/Dimethylsulfoxid, Trifluoracetylchlorid/Dimethylsulfoxid, Toluolsulfonylbromid/Dimethylsulfoxid, Phosphorpentachlorid/Dimethylsulfoxid und Isobutylchlorformiat/Dimethylsulfoxid.

16. Das Verfahren des Anspruchs 13, worin dieses Oxidationsmittel ein Schwefeltrioxid:Pyridincomplex in einem geeigneten Lösungsmittel ist.

17. Das Verfahren des Anspruchs 13, worin die Reaktionstemperatur zwischen etwa 15°C und etwa 30°C liegt.

18. Das Verfahren des Anspruchs 13, worin der geschützte Aminoalkohol [bis(Phenylmethyl)amino]-benzolpropanol ist.

19. Ein Verfahren gemäß Anspruch 1, worin ein geschützter chiraler Alpha-Aminoalkohol der Formel 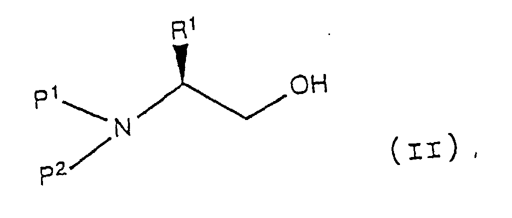
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin jede der vorstehenden Gruppen P¹ und P² substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch vertragliches Salz davon;
hergestellt wird durch Behandlung dieses Aminoalkohols mit einem Alkylierungsmittel.

20. Das Verfahren des Anspruchs 19, worin der Aminoalkohol L-Phenylalaninol ist.

21. Ein Verfahren gemäß Anspruch 1, worin ein geschützter chiraler Alpha-Aminoalkohol der Formel II 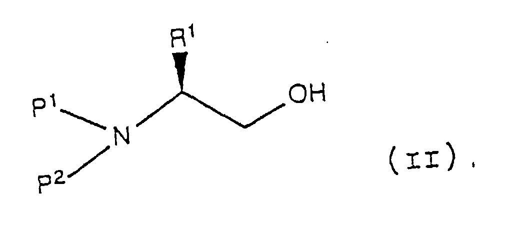
worin P¹ und P² unabhängig voneinander ausgewählt sind aus Acyl, Aralkyl, Alkenyl, Silyl, Aralkoxycarbonyl, Alkoxycarbonyl und Cycloalkenylalkyl;
worin außerdem P¹ und P² zusammengenommen mit dem Stickstoffatom, an das sie gebunden sind, ein heterocyclisches Ringsystem bilden, enthaltend dieses Stickstoffatom als Ringglied;
worin R¹ ausgewählt ist aus Alkyl, Aryl, Cycloalkyl, Cycloalkylalkyl und Aralkyl, die gegebenenfalls substituiert sind mit einer Gruppe ausgewählt aus Alkyl, Halogen, NO₂, OR⁹ und SR⁹, worin R⁹ ausgewählt ist aus Wasserstoff und Alkyl;
und worin jede der vorstehenden Gruppen P¹ und P² substituiert sein kann an einem oder mehreren substituierbaren Positionen mit einem oder mehreren Resten unabhängig voneinander ausgewählt aus Halogen, Alkyl mit C₁-C₈, Alkoxy, Hydroxy, Nitro, Alkenyl, Amino, Alkylamino, Acylamino und Acyl; oder ein pharmazeutisch verträgliches Salz davon;
hergestellt wird durch Behandlung einer Aminosäure mit einem Alkylierungsmittel unter Bildung einer geschützten Aminosäure und Bildung eines geschützten Aminoalkohols durch Behandlung dieser geschützten Aminosäure mit einem Reduktionsmittel in einem geeigneten Lösungsmittel.

22. Das Verfahren des Anspruchs 21, worin das Reduktionsmittel Diisobutylaluminiumhydrid ist.

23. Das Verfahren des Anspruchs 21, worin diese Aminosäure L-Phenylalanin ist.

## Revendications

1. Procédé pour la préparation d'un aminoalcool substitué de formule 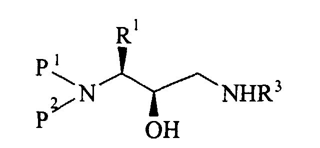
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote auquel ils sont liés pour former un système hétérocyclique contenant ledit atome d'azote en tant que chaînon cyclique;
dans laquelle R¹ est choisi parmi alkyle, aryle, cycloalkyle, cycloalkylalkyle et aralkyle, qui sont éventuellement substitués par un groupe choisi parmi alkyle, halogène, NO₂, OR⁹ et SR⁹, dans lesquels R⁹ est choisi parmi hydrogène et alkyle;
et dans laquelle R³ représente les radicaux hydrogène, alkyle, alcényle, alcynyle, hydroxyalkyle, alcoxyalkyle, cycloalkyle, cycloalkylalkyle, hétérocycloalkyle, hétéroaryle, hétérocycloalkylalkyle, aryle, aralkyle, hétéroaralkyle, aminoalkyle et aminoalkyle mono et disubstitué, dans laquelle lesdits substituants sont choisis parmi les radicaux alkyle, aryle, aralkyle, cycloalkyle, cycloalkylalkyle, hétéroaryle, hétéroaralkyle, hétérocycloalkyle et hétérocycloalkylalkyle, ou dans le cas d'un radical aminoalkyle disubstitué, lesdits substituants forment, avec l'atome d'azote auquel ils sont liés, un radical hétérocycloalkyle ou hétéroaryle;
et dans laquelle l'un quelconque des groupes précédents P¹, P² et R³ peut être substitué en une ou plusieurs positions substituables avec un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables; ledit procédé comprenant l'étape de formation d'un aminoalcool N,N-protégé de formule (II) 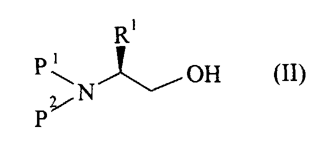 d'oxydation dudit aminoalcool N,N-protégé de formule (II) en un aminoaldéhyde chiral N,N-protégé de 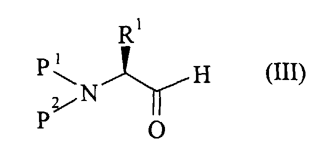 formule (III) et de formation diastéréosélective d'un aminoépoxyde à partir dudit aminoaldéhyde avec un réactif organométallique d'addition d'un groupe méthylène choisi parmi un composé d'halogénométhyllithium dans un solvant approprié et de réaction dudit aminoépoxyde avec une amine de formule R³NH₂, dans laquelle R³ est tel que défini ci-dessus, afin de former ledit aminoalcool substitué.

2. Procédé selon la revendication 1, dans lequel un composé aminoépoxyde de formule I est préparé 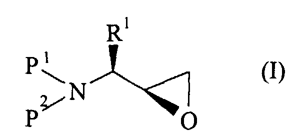
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote de la formule I pour former un système hétérocyclique contenant ledit atome d'azote en tant que chaînon cyclique;
dans laquelle R¹ est choisi parmi alkyle, aryle, cycloalkyle, cycloalkylalkyle et aralkyle, qui sont éventuellement substitués par un groupe choisi parmi alkyle, halogène, NO₂, OR⁹ et SR⁹, dans lesquels R⁹ est choisi parmi hydrogène et alkyle;
et dans laquelle l'un quelconque des groupes précédents P¹ et P² peut être substitué en une ou plusieurs positions substituables avec un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables;
ledit procédé comprenant les étapes de formation d'un aminoalcool N,N-protégé de formule (II) 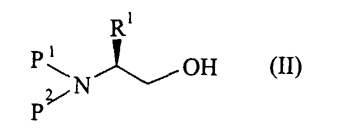 d'oxydation dudit aminoalcool N,N-protégé de formule (II) en un aminoaldéhyde chiral N,N-protégé de formule (III) 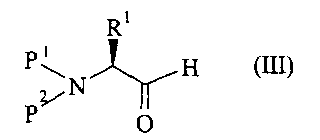 et de formation diastéréosélective d'un aminoépoxyde à partir dudit aminoaldéhyde avec un réactif organométallique d'addition d'un groupe méthylène choisi parmi un composé d'halogénométhyllithium dans un solvant approprié.

3. Procédé selon la revendication 2, dans lequel P¹ et P² sont choisis indépendamment parmi carbobenzoxy, tert-butoxycarbonyle, acétyle, butyryle, benzoyle, isobutoxycarbonyle, allyle, 1,2-bis(diméthylsilyl)éthane, 1,2-bis(diméthylsilylbenzène), benzoyle substitué, trifluoroacétyle, trichloroacétyle, phtaloyle, benzyle, ortho-méthylbenzyle, trityle, 1,2-bis(méthylène)benzène, benzhydryle, phénéthyle, phénpropyle, phényle, naphtalényle, indényle, anthracényle, durényle, 9-(9-phénylfluorényle) et phénanthrényle, dans laquelle en outre P¹ et P² peuvent être pris ensemble pour former avec l'atome d'azote de la formule I un radical choisi parmi phtalimide, succinimide et maléimide et dans laquelle l'un des groupes précédents P¹ et P² peut être substitué en une ou plusieurs positions substituables avec un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables.

4. Procédé selon la revendication 2, dans lequel P¹ et P² sont choisis indépendamment parmi aralkyle, 1,2-bis(méthylène)benzène et aralkyle substitué par un ou plusieurs radicaux choisis indépendamment parmi halogène, alkyle en C₁-C₈, alcoxy, hydroxy, nitro, alcényle, amino, alkylamino, acylamino et acyle; et dans lequel R¹ est aralkyle; ou un de ses sels pharmaceutiquement acceptables.

5. Procédé selon la revendication 1, dans lequel un alpha-aminoépoxyde chiral protégé de formule I 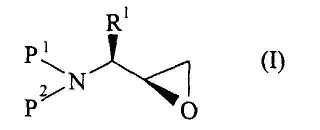
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote de la formule I pour former un système hétérocyclique contenant ledit atome d'azote en tant que chaînon cyclique;
dans laquelle R¹ est choisi parmi alkyle, aryle, cycloalkyle, cycloalkylalkyle et aralkyle, qui sont éventuellement substitués par un groupe choisi parmi alkyle, halogène, NO₂, OR⁹ et SR⁹, dans lesquels R⁹ est choisi parmi hydrogène et alkyle;
et dans laquelle l'un quelconque des groupes précédents P¹ et P² peut être substitué en une ou plusieurs positions substituables avec un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables;
est préparé d'une manière diastéréosélective et énantiosélective par traitement d'un substrat aminoaldéhyde protégé avec un réactif organométallique d'addition d'un groupe méthylène dans un solvant approprié.

6. Procédé selon la revendication 5, dans lequel le réactif organométallique d'addition d'un groupe méthylène est un halogénométhyllithium généré in situ.

7. Procédé selon la revendication 6, dans lequel on ajoute à l'aminoaldéhyde au moins une quantité équimolaire du réactif organométallique d'addition d'un groupe méthylène.

8. Procédé selon la revendication 5, dans lequel l'halogénométhyllithium est formé par addition d'un réactif organolithié à un dihalogénométhane.

9. Procédé selon la revendication 5, dans lequel le dihalogénométhane est choisi parmi le bromochlorométhane, le chloroiodométhane, le dibromométhane, le diiodométhane et le bromofluorométhane.

10. Procédé selon la revendication 5, dans lequel l'halogénométhyllithium est ajouté à l'aminoaldéhyde à une température dans une gamme comprise entre environ -80°C et environ 0°C.

11. Procédé selon la revendication 5, dans lequel l'halogénométhyllithium est ajouté à l'aminoaldéhyde à une température dans la gamme comprise entre environ -40°C et -15°C.

12. Procédé diastéréosélectif pour la préparation des alpha-aminoépoxydes chiraux protégés selon la revendication 5, dans lequel l'aminoaldéhyde est l'alpha-S-[bis(phénylméthyl)amino]benzènepropanaldéhyde.

13. Procédé selon la revendication 1, dans lequel on prépare un alpha-aminoaldéhyde protégé de formule III: 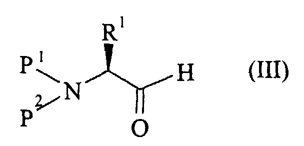
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote de la formule III pour former un système hétérocyclique contenant ledit atome d'azote en tant que chaînon cyclique;
et dans laquelle R¹ est choisi parmi alkyle, aryle, cycloalkyle, cycloalkylalkyle et aralkyle, qui sont éventuellement substitués par un groupe choisi parmi alkyle, halogène, NO₂, OR⁹ et SR⁹, dans lesquels R⁹ est choisi parmi hydrogène et alkyle;
et dans laquelle l'un quelconque des groupes précédents P¹ et P² peut être substitué en une ou plusieurs positions substituables avec un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables;
par traitement à une température comprise entre 0°C et 30°C d'un aminoalcool protégé avec un agent d'oxydation.

14. Procédé selon la revendication 13, dans lequel on ajoute au moins une quantité équimolaire dudit agent d'oxydation à l'aminoalcool protégé.

15. Procédé selon la revendication 13, dans lequel ledit agent d'oxydation est choisi parmi le complexe trioxyde de soufre:pyridine, le chlorure d'acétyle/diméthylsulfoxyde, l'anhydride acétique/diméthylsulfoxyde, le chlorure de trifluoroacétyle/diméthylsulfoxyde, le bromure de toluènesulfonyle/diméthylsulfoxyde, le pentachlorure de phosphore/diméthylsulfoxyde et le chloroformiate d'isobutyle/diméthylsulfoxyde.

16. Procédé selon la revendication 13, dans lequel ledit agent d'oxydation est le complexe trioxyde de soufre:pyridine dans un solvant approprié.

17. Procédé selon la revendication 13, dans lequel la température de la réaction est comprise entre environ 15°C et environ 30°C.

18. Procédé selon la revendication 13, dans lequel l'aminoalcool protégé est le [bis(phénylméthyl)aminol]benzènepropanol.

19. Procédé selon la revendication 1, dans lequel on prépare un alpha-aminoalcool chiral protégé de formule: 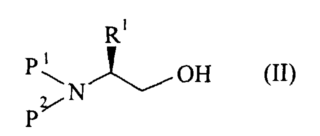
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote de la formule II pour former un système hétérocyclique contenant ledit atome d'azote en tant que chaînon cyclique;
et dans laquelle R¹ est choisi parmi alkyle, aryle, cycloalkyle, cycloalkylalkyle et aralkyle, qui sont éventuellement substitués en une ou plusieurs positions substituables par un groupe choisi parmi alkyle, halogène, NO₂, OR⁹ et SR⁹, dans lesquels R⁹ est choisi parmi hydrogène et alkyle;
et dans laquelle l'un quelconque des groupes précédents P¹ et P² peut être substitué par un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables;
par traitement dudit aminoalcool avec un agent d'alkylation.

20. Procédé selon la revendication 19, dans lequel l'aminoalcool est le L-phénylalaninol.

21. Procédé selon la revendication 1, dans lequel on prépare un alpha-aminoalcool chiral protégé de formule II: 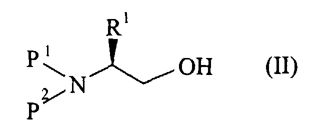
dans laquelle P¹ et P² sont choisis indépendamment parmi acyle, aralkyle, alcényle, silyle, aralcoxycarbonyle, alcoxycarbonyle et cycloalcénylalkyle;
dans laquelle en outre P¹ et P² peuvent être pris ensemble avec l'atome d'azote de la formule II pour former un système hétérocyclique contenant ledit atome d'azote en tant que chaînon cyclique;
et dans laquelle R¹ est choisi parmi alkyle, aryle, cycloalkyle, cycloallcylalkyle et aralkyle, qui sont éventuellement substitués en une ou plusieurs positions substituables par un groupe choisi parmi alkyle, halogène, NO₂, OR⁹ et SR⁹, dans lesquels R⁹ est choisi parmi hydrogène et alkyle;
et dans laquelle l'un quelconque des groupes précédents P¹ et P² peut être substitué par un ou plusieurs radicaux choisis indépendamment parmi halogène, alkyle en C₁-C₈, alcoxy, hydroxy, nitro, alcényle, amino, alkylamino, acylamino et acyle; ou un de ses sels pharmaceutiquement acceptables;
par traitement d'un acide aminé avec un agent d'alkylation afin de former un acide aminé protégé et par formation d'un aminoalcool protégé par traitement dudit acide aminé protégé avec un agent réducteur dans un solvant approprié.

22. Procédé selon la revendication 21, dans lequel l'agent réducteur est l'hydrure de diisobutylaluminium.

23. Procédé selon la revendication 21, dans lequel ledit acide aminé est la L-phénylalanine.
